# EUROPEAN PATENT APPLICATION

(11) **EP 4 386 003 A1**
(43) Date of publication of application: **19.06.2024**
(21) Application number: 22855431.7
(22) Date of filing: 09.08.2022
(51) Int. Cl.: C07K 16/28, C07K 16/46, C12N 15/13, A61K 39/395, A61P 35/00

(54) **ANTI-TIGIT ANTIBODY AND USE THEREOF**

(30) Priority: 09.08.2021 CN 202110907583
(71) Applicant: Biotheus Inc., Tangjiawan Town, Xiangzhou District Zhuhai, Guangdong 519080 (CN)
(72) Inventor: ZHAI, Tianhang, Zhuhai, Guangdong 519080 (CN); HUANG, Weifeng, Zhuhai, Guangdong 519080 (CN); DAI, Shuang, Zhuhai, Guangdong 519080 (CN); XU, Yingda, Zhuhai, Guangdong 519080 (CN); BUKOWSKI, Jon, Lebanon, NH 03766 (US); SCHUTZ, Kevin, Lebanon, NH 03766 (US); HEMMERLEIN, Megan, Lebanon, NH 03766 (US)
(74) Representative: Bottero, Carlo
(86) International application number: PCT/CN2022/111145
(87) International publication number: WO 2023/016450

(57) **Abstract**

An antibody specifically binding to TIGIT or an antigen-binding fragment thereof can effectively block the binding of TIGIT to a ligand thereof and release the inhibitory effect of the ligand of TIGIT on a downstream signal of TIGIT. The present invention further relates to a composition containing the antibody or antigen-binding fragment thereof, a nucleic acid coding the antibody or the antigen-binding fragment thereof, a host cell containing the nucleic acid, and a related use. In addition, the present invention also relates to treatment and diagnosis uses of the antibody or the antigen-binding fragment thereof.

## Description

### Technical Field

The present invention relates to an antibody or antigen-binding fragment thereof that specifically binds TIGIT and a composition containing the antibody or antigen-binding fragment thereof. Furthermore, the present invention relates to a nucleic acid molecule encoding the antibody or antigen-binding fragment thereof and a host cell comprising the nucleic acid molecule, and related uses thereof. Furthermore, the present invention relates to the therapeutic and diagnostic uses of the antibody or antigen-binding fragment thereof.

### Background Art

T cell immunoreceptor with Ig and ITIM domains (TIGIT, also known as WUCAM, Vstm3, VSIG9) is a new type of immunosuppressive receptor expressed by activated CD8⁺ T and CD4⁺ T cells, natural killer (NK) cells, regulatory T cells (Tregs) and follicular helper T cells.

TIGIT participates in a complex regulatory network in tumor immunity, involving multiple immunosuppressive receptors (e.g., CD96/TACTILE, CD112R/PVRIG), a competitive costimulatory receptor (DNAM-1/CD226), and multiple ligands (e.g., CD155 (PVR/NECL-5), CD112 (Nectin-2/PVRL2)). DNAM-1, TIGIT and CD96 are expressed on T cells and NK cells and share CD155 as a ligand. In preclinical mouse tumor models, TIGIT deficiency delayed the growth of subcutaneous B16F10 and MC38 tumors (Kurtulus S, Sakuishi K, Ngiow SF, et al. TIGIT predominantly regulates the immune response via regulatory T cells. J Clin Invest. 2015 Nov 2;125(11):4053-62.). In addition, when mice were inoculated with melanoma B16 cells, very few TIGIT-/- mice developed lung metastasis compared with mice with normal expression of the TIGIT gene, and the overall survival of tumor-bearing TIGIT-/- mice was significantly prolonged (Zhang Q, Bi J, Zheng X, et al. Blockade of the checkpoint receptor TIGIT prevents NK cell exhaustion and elicits potent anti-tumor immunity. Nat Immunol. 2018 Jul;19(7):723-732.). These results indicate that TIGIT plays a key role in tumor immunity as an immunosuppressive receptor.

The role of TIGIT in tumor immunosuppression is similar to that of PD-1/PD-L1. Based on the mechanism of TIGIT-mediated tumor immune response, researchers use TIGIT blocking monoclonal antibodies for therapeutic intervention, so as to enhance the activity of T or NK cells, and exert anti-tumor effects (Guillerey C, Harjunpaa H, Carrie N, et al. TIGIT immune checkpoint blockade restores CD8(+) T-cell immunity against multiple myeloma. Blood 2018, 132, 1689-1694.). Another study reported that anti-TIGIT monoclonal antibodies could delay the growth of subcutaneous CT26 tumors and methylcholanthrene (MCA)-induced fibromas, and could also protect mice from experimental metastasis of 4T1 or B16 tumors (Zhang Q, Bi J, Zheng X, et al. Blockade of the checkpoint receptor TIGIT prevents NK cell exhaustion and elicits potent anti-tumor immunity. Nat Immunol. 2018 Jul;19(7):723-732.).

In view of the multiple roles of TIGIT in immune responses, TIGIT is considered as an attractive target for tumor immunotherapy. Therefore, there is a need in the art to develop new TIGIT antibodies for disease treatment, especially cancer treatment.

### Contents of the present invention

After extensive research, the inventors of the present application screened and obtained a series of fully human antibodies against TIGIT, which have high binding affinity to TIGIT and can effectively block the binding of TIGIT to its ligand CD 155/CD 112, thereby reducing or eliminating the inhibitory signals transmitted to the cell, and the administration of the antibody of the present invention can significantly inhibit tumor growth in animal models. Based on this, the present application also provides a composition containing the antibody or antigen-binding fragment thereof, a nucleic acid molecule encoding the antibody or antigen-binding fragment thereof, a host cell containing the same, and related uses thereof.

### ADI-55796

Therefore, in a first aspect, the present application provides an antibody or antigen-binding fragment thereof capable of specifically binding to TIGIT, the antibody or antigen-binding fragment thereof comprises:
a VH CDR1 or variant thereof, a VH CDR2 or variant thereof, and a VH CDR3 or variant thereof comprised in the heavy chain variable region (VH) as set forth in SEQ ID NO: 4; and/or, a VL CDR1 or variant thereof, a VL CDR2 or variant thereof, and a VL CDR3 or variant thereof comprised in the light chain variable region (VL) as set forth in SEQ ID NO: 8;
wherein, the variant has a substitution, deletion or addition of one or several amino acids (e.g., a substitution (such as conservative substitution), deletion or addition of 1, 2 or 3 amino acids) as compared to the sequence from which it is derived. In certain embodiments, the substitution is a conservative substitution.

In certain embodiments, the antibody or antigen-binding fragment thereof comprises: three CDRs comprised in the heavy chain variable region (VH) as set forth in SEQ ID NO: 4; and/or, three CDRs comprised in the light chain variable region (VL) as set forth in SEQ ID NO: 8.

In certain embodiments, the three CDRs comprised in the VH and/or the three CDRs comprised in the VL are defined by the Kabat, IMGT or Chothia numbering system.

In certain embodiments, the antibody or antigen-binding fragment thereof comprises:
the following three complementarity-determining regions (CDRs) of the heavy chain variable region (VH): a VH CDR1 having the sequence as set forth in SEQ ID NO: 1, a VH CDR2 having the sequence as set forth in SEQ ID NO: 2, and a VH CDR3 having the sequence as set forth in SEQ ID NO: 3; and/or, the following three complementarity-determining regions (CDRs) of the light chain variable region (VL): a VL CDR1 having the sequence as set forth in SEQ ID NO: 5, a VL CDR2 having the sequence as set forth in SEQ ID NO: 6, a VL CDR3 having the sequence as set forth in SEQ ID NO: 7;
wherein, the CDRs are defined by the IMGT numbering system.

In certain embodiments, the antibody or antigen-binding fragment thereof further comprises a framework region of a human immunoglobulin.

In certain embodiments, the antibody or antigen-binding fragment thereof comprises a framework region comprised in an amino acid sequence encoded by a human antibody germline gene. In certain embodiments, the antibody or antigen-binding fragment thereof comprises: a heavy chain framework region comprised in an amino acid sequence encoded by a human heavy chain germline gene, and/or a light chain framework region comprised in an amino acid sequence encoded by a human light chain germline gene.

In certain embodiments, the antibody or antigen-binding fragment thereof comprises: a VH comprising the sequence as set forth in SEQ ID NO: 4 or variant thereof, and/or a VL comprising the sequence as set forth in SEQ ID NO: 8 or variant thereof;
wherein, the variant has a substitution, deletion or addition of one or several amino acids (e.g., a substitution, deletion or addition of 1, 2, 3, 4 or 5 amino acids), or has a sequence identity of at least 80%, at least 85%, at least 90%, at least 91%, at least 92%, at least 93%, at least 94%, at least 95%, at least 96%, at least 97%, at least 98%, at least 99% or 100%, as compared to the sequence from which it is derived. In certain embodiments, the substitution is a conservative substitution.

In certain embodiments, the antibody or antigen-binding fragment thereof comprises a VH as set forth in SEQ ID NO: 4, and/or a VL as set forth in SEQ ID NO: 8.

In certain embodiments, the antibody or antigen-binding fragment thereof is capable of specifically binding human TIGIT and/or blocking the binding of human TIGIT to its ligand, for example, the activity of the binding and/or the activity of the blocking is superior to that of Tiragolumab.

In certain embodiments, the antibody or antigen-binding fragment thereof is capable of specifically binding mouse TIGIT and/or blocking the binding of mouse TIGIT to its ligand.

In certain preferred embodiments, the antibody or antigen-binding fragment thereof has cross-reactivity with human, cynomolgus and/or mouse TIGIT, for example, has cross-reactivity with human, cynomolgus and mouse TIGIT.

### ADI-55812

In a second aspect, the present application provides an antibody or antigen-binding fragment thereof capable of specifically binding TIGIT, the antibody or antigen-binding fragment thereof comprises:
a VH CDR1 or variant thereof, a VH CDR2 or variant thereof, and a VH CDR3 or variant thereof comprised in the heavy chain variable region (VH) as set forth in SEQ ID NO: 12; and/or, a VL CDR1 or variant thereof, a VL CDR2 or variant thereof, and a VL CDR3 or variant thereof comprised in the light chain variable region (VL) as set forth in SEQ ID NO: 16;
wherein, the variant has a substitution, deletion or addition of one or several amino acids (e.g., a substitution (such as conservative substitution), deletion or addition of 1, 2 or 3 amino acids) as compared to the sequence from which it is derived. In certain embodiments, the substitution is a conservative substitution.

In certain embodiments, the antibody or antigen-binding fragment thereof comprises: three CDRs comprised in the heavy chain variable region (VH) as set forth in SEQ ID NO: 12; and/or, three CDRs comprised in the light chain variable region (VL) as set forth in SEQ ID NO: 16.

In certain embodiments, the three CDRs comprised in the VH and/or the three CDRs comprised in the VL are defined by the Kabat, IMGT or Chothia numbering system.

In certain embodiments, the antibody or antigen-binding fragment thereof comprises:
the following three complementarity-determining regions (CDRs) of the heavy chain variable region (VH): a VH CDR1 having the sequence as set forth in SEQ ID NO: 9, a VH CDR2 having the sequence as set forth in SEQ ID NO: 10, and a VH CDR3 having the sequence as set forth in SEQ ID NO: 11; and/or, the following three complementarity-determining regions (CDRs) of the light chain variable region (VL): a VL CDR1 having the sequence as set forth in SEQ ID NO: 13, a VL CDR2 having the sequence as set forth in SEQ ID NO: 14, a VL CDR3 having the sequence as set forth in SEQ ID NO: 15;
wherein, the CDRs are defined by the IMGT numbering system.

In certain embodiments, the antibody or antigen-binding fragment thereof further comprises a framework region of a human immunoglobulin.

In certain embodiments, the antibody or antigen-binding fragment thereof comprises a framework region comprised in an amino acid sequence encoded by a human antibody germline gene. In certain embodiments, the antibody or antigen-binding fragment thereof comprises: a heavy chain framework region comprised in an amino acid sequence encoded by a human heavy chain germline gene, and/or a light chain framework region comprised in an amino acid sequence encoded by a human light chain germline gene.

In certain embodiments, the antibody or antigen-binding fragment thereof comprises: a VH comprising the sequence as set forth in SEQ ID NO: 12 or variant thereof, and/or a VL comprising the sequence as set forth in SEQ ID NO: 16 or variant thereof;
wherein, the variant has a substitution, deletion or addition of one or several amino acids (e.g., a substitution, deletion or addition of 1, 2, 3, 4 or 5 amino acids), or has a sequence identity of at least 80%, at least 85%, at least 90%, at least 91%, at least 92%, at least 93%, at least 94%, at least 95%, at least 96%, at least 97%, at least 98%, at least 99% or 100%, as compared to the sequence from which it is derived. In certain embodiments, the substitution is a conservative substitution.

In certain embodiments, the antibody or antigen-binding fragment thereof comprises a VH as set forth in SEQ ID NO: 12, and/or a VL as set forth in SEQ ID NO: 16.

In certain embodiments, the antibody or antigen-binding fragment thereof is capable of specifically binding human TIGIT and/or blocking the binding of human TIGIT to its ligand, for example, the activity of the binding and/or the activity of the blocking is superior to that of Tiragolumab.

In certain preferred embodiments, the antibody or antigen-binding fragment thereof has cross-reactivity with human and cynomolgus TIGIT.

In certain embodiments, the antibody or antigen-binding fragment thereof of the first or second aspect of the present invention further comprises a constant region derived from a human immunoglobulin.

In certain embodiments, the heavy chain of the antibody or antigen-binding fragment thereof comprises the heavy chain constant region (CH) of a human immunoglobulin or variant thereof, and the variant has a substitution, deletion or addition of one or several amino acids (e.g., a substitution, deletion or addition of up to 20, up to 15, up to 10, or up to 5 amino acids; for example, a substitution, deletion or addition of 1, 2, 3, 4 or 5 amino acids) as compared to the sequence from which it is derived; and/or,
the light chain of the antibody or antigen-binding fragment thereof comprises the light chain constant region (CL) of a human immunoglobulin or variant thereof, and the variant has a conservative substitution of up to 20 amino acids (e.g., a conservative substitution of up to 15, up to 10, or up to 5 amino acids; such as a conservative substitution of 1, 2, 3, 4 or 5 amino acids) as compared to the sequence from which it is derived.

In some embodiments, the variant of the heavy chain constant region (CH) may have a conservative substitution of one or more amino acids as compared to the sequence from which it is derived. In such embodiments, the variant of the heavy chain constant region (CH) may have the same or substantially the same effector function as compared to the wild-type sequence from which it is derived.

In other embodiments, the variant of the heavy chain constant region (CH) may comprise one or more amino acid mutations or chemical modifications to alter one or more of the following properties of the antibody of the present invention: Fc receptor binding, antibody glycosylation, number of cysteine residues, effector cell function, or complement function, etc. Effector function can be altered by replacing at least one amino acid residue in the constant region of the antibody with a different residue or by chemical modification to produce a functional change, for example, changing the affinity of the antibody to an effector ligand (e.g., FcR or complement C1q) may alter (e.g., reduce or enhance) effector function. The Fc region of the antibody mediates several important effector functions, such as ADCC, phagocytosis, CDC, etc.

In certain embodiments, the antibody or antigen-binding fragment thereof possesses ADCC activity. In certain embodiments, the antibody or antigen-binding fragment thereof comprises a mutated (e.g., amino acid substitution) or chemically modified Fc region, wherein the mutated or chemically modified Fc region provides enhanced ADCC activity. In certain embodiments, the antibody or antigen-binding fragment thereof is produced by expression in a host cell with low or no fucosylation activity, and the antibody produced has low or no fucosylation, which enhances its ADCC activity. Such host cells may be a mammalian cell, such as CHO cell, which is lack of a gene encoding a fucosyltransferase (e.g., FUT8).

In certain embodiments, the heavy chain constant region is an IgG heavy chain constant region, such as IgG1, IgG2, IgG3, or IgG4 heavy chain constant region, such as IgG1 heavy chain constant region. In certain embodiments, the antibody or antigen-binding fragment thereof is selected from IgG, such as IgG1, IgG2, IgG3, or IgG4. In certain exemplary embodiments, the heavy chain of the antibody or antigen-binding fragment thereof comprises a heavy chain constant region as set forth in SEQ ID NO: 17 or SEQ ID NO: 18.

In certain embodiments, the light chain of the antibody or antigen-binding fragment thereof comprises a light chain constant region (e.g., a constant region of κ or λ chain) derived from a human immunoglobulin. In certain exemplary embodiments, the light chain of the antibody or antigen-binding fragment thereof comprises a light chain constant region as set forth in SEQ ID NO: 22.

In certain embodiments, the antigen-binding fragment is selected from the group consisting of Fab, Fab', (Fab')₂, Fv, disulfide-linked Fv, scFv, diabody, and single domain antibody (sdAb); and/or, the antibody is a chimeric antibody, a bispecific antibody or a multispecific antibody.

The antibody of the present invention can be prepared by various methods known in the art, such as by genetic engineering and recombinant technology. For example, DNA molecules encoding the heavy chain and light chain of the antibody of the present invention are obtained by chemical synthesis or PCR amplification. The resulting DNA molecule is inserted into an expression vector and then transfected into host cells. Then, the transfected host cells are cultured under specific conditions to express the antibody of the present invention.

The antigen-binding fragments of the present invention can be obtained by hydrolyzing intact antibody molecules (see Morimoto et al., J. Biochem. Biophys. Methods 24:107-117 (1992) and Brennan et al., Science 229:81 (1985)). Besides, these antigen-binding fragments can also be produced directly from recombinant host cells (reviewed in Hudson, Curr. Opin. Immunol. 11: 548-557 (1999); Little et al., Immunol. Today, 21: 364-370 (2000)). For example, Fab' fragments can be obtained directly from host cells; Fab' fragments can be chemically coupled to form F(ab')₂ fragments (Carter et al., Bio/Technology, 10: 163-167 (1992)). In addition, Fv, Fab or F(ab')₂ fragments can also be directly isolated from the cell culture of the recombinant host cell. Those of ordinary skill in the art are well aware of other techniques for preparing such antigen-binding fragments.

### Nucleic acid molecules

In another aspect, the present application provides an isolated nucleic acid molecule, which encodes the antibody or antigen-binding fragment thereof as described above, or heavy chain variable region and/or light chain variable region thereof.

In certain embodiments, the isolated nucleic acid molecule comprises a first nucleotide sequence encoding a heavy chain or heavy chain variable region of the antibody or antigen-binding fragment thereof of the present invention and a second nucleotide sequence encoding a light chain or light chain variable region of the antibody or antigen-binding fragment thereof, wherein the first nucleotide sequence and the second nucleotide sequence are present on the same or different isolated nucleic acid molecules. When the first nucleotide sequence and the second nucleotide sequence are present on different isolated nucleic acid molecules, the isolated nucleic acid molecule of the present invention includes a first nucleic acid molecule comprising the first nucleotide sequence and a second nucleic acid molecule comprising the second nucleotide sequence.

### Vectors

In another aspect, the present application provides a vector, which comprises the nucleic acid molecule as described above. In certain embodiments, the vector is a cloning vector or an expression vector.

In certain embodiments, the vector comprises a first nucleotide sequence encoding a heavy chain or heavy chain variable region of the antibody or antigen-binding fragment thereof of the present invention and a second nucleotide sequence encoding a light chain or light chain variable region of the antibody or antigen-binding fragment thereof, wherein the first nucleotide sequence and the second nucleotide sequence are present on the same or different vectors. When the first nucleotide sequence and the second nucleotide sequence are present on different vectors, the vector of the present invention includes a first vector comprising the first nucleotide sequence and a second vector comprising the second nucleotide sequence.

### Host cells

In another aspect, the present application also provides a host cell, which comprises the nucleic acid molecule or vector as described above. Such host cell includes, but is not limited to, prokaryotic cell such as bacterial cell (e.g., *E. coli* cell), and eukaryotic cell such as fungal cell (e.g., yeast cell), insect cell, plant cell, and animal cell (e.g., mammalian cell, for example, mouse cell, human cell, etc.).

In certain embodiments, the host cell has low or no fucosylation activity. In certain embodiments, the host cell can be a mammalian cell, such as a CHO cell, which is lack of a gene encoding a fucosyltransferase (e.g., FUT8).

### Preparation method

In another aspect, the present application provides a method for preparing the antibody or antigen-binding fragment thereof as described above, which comprises culturing the host cell as described above under a condition that allows expression of the antibody or antigen-binding fragment thereof, and recovering the antibody or antigen-binding fragment thereof from a cell culture of the host cell.

In certain embodiments, the host cell has low or no fucosylation activity, thereby producing an antibody with low or no fucosylation, and this enhances the ADCC activity of the antibody produced therein. In certain embodiments, the host cell can be a mammalian cell, such as a CHO cell, which is lack of a gene encoding a fucosyltransferase (e.g., FUT8).

### Pharmaceutical composition

In another aspect, the present application provides a pharmaceutical composition, which comprises the antibody or antigen-binding fragment thereof, the isolated nucleic acid molecule, the vector or the host cell as described above, and a pharmaceutically acceptable carrier and/or excipient.

In certain embodiments, the pharmaceutical composition further comprises an additional immune checkpoint inhibitor.

In certain embodiments, the pharmaceutical composition further comprises an anti-PD-1 antibody or an anti-PD-L1 antibody.

In certain exemplary embodiments, the pharmaceutically acceptable carrier and/or excipient comprises a sterile injectable liquid (e.g., an aqueous or non-aqueous suspension or solution). In certain exemplary embodiments, such sterile injectable liquid is selected from the group consisting of water for injection (WFI), bacteriostatic water for injection (BWFI), sodium chloride solution (e.g., 0.9% (w/v) NaCl), dextrose solution (e.g., 5% glucose), surfactant-containing solution (e.g., a solution containing 0.01% polysorbate 20), pH buffer solution (e.g., phosphate buffer solution), Ringer's solution and any combination thereof.

### Uses

In another aspect, the present application provides a use of the antibody or antigen-binding fragment thereof, the isolated nucleic acid molecule, the vector, the host cell or the pharmaceutical composition as described above in the manufacture of a medicament, and the medicament is used for:
(1) increasing an immune cell activity in vitro or in a subject (e.g., a human);
(2) enhancing an immune response in a subject (e.g., a human);
(3) preventing and/or treating a tumor in a subject (e.g., a human); or
(4) preventing and/treating an infection in a subject (e.g., a human).

In certain embodiments, the immune cell is a T cell, and/or a NK cell.

In certain embodiments, the immune response is a T cell- or NK cell-mediated immune response.

In certain embodiments, the tumor involves a TIGIT-positive infiltrating T cell and/or NK cell, and/or involves a TIGIT ligand (e.g., CD155 and/or CD112)-positive tumor cell.

In certain embodiments, the tumor is selected from a solid tumor or a hematological tumor (e.g., leukemia, lymphoma, myeloma).

In certain embodiments, the tumor is selected from the group consisting of colorectal cancer, colon cancer, bladder cancer, breast cancer, uterine/cervical cancer, ovarian cancer, prostate cancer, testicular cancer, esophageal cancer, gastrointestinal cancer, pancreatic cancer, renal cancer, head and neck cancer, lung cancer, stomach cancer, germ cell cancer, bone cancer, liver cancer, thyroid cancer, skin cancer, central nervous system cancer, lymphoma, leukemia, myeloma, sarcoma, and melanoma.

In certain embodiments, the infection is selected from the group consisting of viral infection, bacterial infection, fungal infection, and parasitic infection.

In certain embodiments, the subject is a mammal, such as a human, a cynomolgus monkey, or a mouse.

In certain embodiments, the antibody or antigen-binding fragment thereof, the isolated nucleic acid molecule, the vector, the host cell, or the pharmaceutical composition is used alone or in combination with an additional pharmaceutically active agent. In certain embodiments, the additional pharmaceutically active agent is an additional immune checkpoint inhibitor.

In certain embodiments, the antibody or antigen-binding fragment thereof, the isolated nucleic acid molecule, the vector, the host cell, or the pharmaceutical composition is used in combination with an anti-PD-1 antibody or an anti-PD-L1 antibody.

In another aspect, the present application provides a method for enhancing an immune response or preventing and/or treating a tumor or infection in a subject, which comprises: administering to the subject in need thereof an effective amount of the antibody or antigen-binding fragment thereof or the pharmaceutical compositions as described above.

In certain embodiments, the tumor involves a TIGIT-positive infiltrating T cell and/or NK cell, and/or involves a TIGIT ligand (e.g., CD155 and/or CD112)-positive tumor cell.

In certain embodiments, the tumor is selected from a solid tumor or a hematological tumor (e.g., leukemia, lymphoma, myeloma).

In certain embodiments, the tumor is selected from the group consisting of colorectal cancer, colon cancer, bladder cancer, breast cancer, uterine/cervical cancer, ovarian cancer, prostate cancer, testicular cancer, esophageal cancer, gastrointestinal cancer, pancreatic cancer, renal cancer, head and neck cancer, lung cancer, stomach cancer, germ cell cancer, bone cancer, liver cancer, thyroid cancer, skin cancer, central nervous system cancer, lymphoma, leukemia, myeloma, sarcoma, and melanoma.

In certain embodiments, the infection is selected from the group consisting of viral infection, bacterial infection, fungal infection, and parasitic infection.

In certain embodiments, the subject is a mammal, such as a human.

The antibody or antigen-binding fragment thereof or the pharmaceutical composition of the present application can be formulated into any dosage form known in the medical field, for example, tablet, pill, suspension, emulsion, solution, gel, capsule, powder, granule, elixir, tablet, suppository, injection (including injectable solution, sterile powder for injection and concentrated solution for injection), inhalant, spray, etc. The preferred dosage form depends on the intended mode of administration and therapeutic use. The antibody or antigen-binding fragment thereof or pharmaceutical composition of the present invention should be sterile and stable under the conditions of production and storage. One preferred dosage form is an injection. Such injection may be a sterile injectable solution. For example, the sterile injectable solution may be prepared by incorporating in an appropriate solvent the requisite dose of the antibody or antigen-binding fragment thereof of the present invention, and, optionally, other desired ingredients (including, but not limited to, pH adjuster, surfactant, adjuvant, ionic strength enhancer, isotonic agent, preservative, diluent, or any combination thereof), followed by filter sterilization. Additionally, the sterile injectable solution may be prepared as a sterile lyophilized powder (e.g., by vacuum drying or freeze drying) for ease of storage and use. Such sterile lyophilized powder can be dispersed in a suitable carrier before use, such as water for injection (WFI), bacteriostatic water for injection (BWFI), sodium chloride solution (e.g., 0.9% (w/v) NaCl), glucose solution (e.g., 5% glucose), surfactant-containing solution (e.g., a solution containing 0.01% polysorbate 20), pH buffer solution (e.g., phosphate buffer solution), Ringer's solution and any combination thereof.

The antibody or antigen-binding fragment thereof of the present application, or the pharmaceutical compositions of the present invention may be administered by any suitable method known in the art, including but not limited to, oral, buccal, sublingual, ophthalmic, local, parenteral, rectal, intrathecal, intra-cisternal, inguinal, intravesical, topical (e.g., powder, ointment, or drop), or nasal route. However, for many therapeutic uses, the preferred route/mode of administration is parenteral administration (e.g., intravenous or bolus injection, subcutaneous injection, intraperitoneal injection, intramuscular injection). Those skilled in the art will understand that the route and/or mode of administration will vary depending on the intended purpose. In certain embodiments, the antibody or antigen-binding fragment thereof or the pharmaceutical composition of the present invention is administered by intravenous injection or bolus injection.

### Conjugate

In another aspect, the present application provides a conjugate, which comprises the antibody or antigen-binding fragment thereof as described above, and a detectable label linked to the antibody or antigen-binding fragment thereof.

In certain embodiments, the detectable label is selected from the group consisting of enzyme (e.g., horseradish peroxidase or alkaline phosphatase), chemiluminescent reagent (e.g., acridinium ester, luminol and derivative thereof, or ruthenium derivative), fluorescent dye (e.g., fluorescein or fluorescent protein), radionuclide and biotin.

### Kit

In another aspect, the present application provides a kit, which comprises the antibody or antigen-binding fragment thereof or the conjugate as described above.

In certain embodiments, the kit comprises the conjugate as described above.

In certain embodiments, the kit comprises the antibody or antigen-binding fragment thereof as described above, and a second antibody that specifically recognizes the antibody or antigen-binding fragment thereof. In certain embodiments, the second antibody further comprises a detectable label, such as an enzyme (e.g., horseradish peroxidase or alkaline phosphatase), a chemiluminescent reagent (e.g., acridinium esters, luminol and derivative thereof, or ruthenium derivative), fluorescent dye (e.g., fluorescein or fluorescent protein), radionuclide or biotin.

In another aspect, the present application provides a method for detecting the presence or level of TIGIT in a sample, which comprises using the antibody or antigen-binding fragment thereof as described above or the conjugate as described above. In certain embodiments, the method is used for a therapeutic purpose, a diagnostic purposes, or a non-therapeutic non-diagnostic purpose.

In certain embodiments, the method is an immunological assay, such as a Western blot, an enzyme immunoassay (e.g., ELISA), a chemiluminescent immunoassay, a fluorescent immunoassay, or a radioimmunoassay.

In certain embodiments, the method comprises using the conjugate as described above.

In certain embodiments, the method comprising using the antibody or antigen-binding fragment thereof as described above, and the method further comprises using a second antibody carrying a detectable label (e.g., an enzyme (e.g., horseradish peroxidase or alkaline phosphatase), chemiluminescent reagent (e.g., acridinium ester, luminol and derivative thereof, or ruthenium derivative), fluorescent dye (e.g., fluorescein or fluorescent protein), radionuclide, or biotin) to detect the antibody or antigen-binding fragment thereof.

In certain embodiments, the method comprises: (1) contacting the sample with the antibody or antigen-binding fragment thereof of the present invention; (2) detecting the formation of an antigen-antibody immune complex or detecting an amount of the immune complex. The formation of the immune complex indicates the presence of TIGIT or TIGIT-expressing cells.

In another aspect, the present application also provides a use of the antibody or antigen-binding fragment thereof or the conjugate as described above in the manufacture of a detection reagent for detecting the presence or level of TIGIT in a sample.

In certain embodiments, the detection reagent detects the presence or level of TIGIT in the sample by the method as described above.

In certain embodiments, the sample is a cell sample (e.g., immune cell) from a subject (e.g., a mammal, preferably a human, a cynomolgus monkey, or a mouse).

### Definition of Terms

In the present invention, unless otherwise stated, scientific and technical terms used herein have the meanings commonly understood by those skilled in the art. Moreover, the virology, biochemistry, and immunology laboratory procedures used in herein are routine procedures widely used in the corresponding fields. Meanwhile, in order to better understand the present invention, definitions and explanations of relevant terms are provided below.

When the terms "for example," "such as," "e.g.," "comprising," "including," or variations thereof are used herein, these terms will not be considered limiting terms and will instead be interpreted to mean "but without limitation" or "without limitation".

Unless otherwise indicated herein or clearly contradicted by context, the terms "a" and "an" as well as "the" and similar referents in the context for describing the present invention (especially in the context of the following claims) are to be construed to cover singular and plural.

As used herein, the term "antibody" refers to an immunoglobulin molecule typically composed of two pairs of polypeptide chains, each pair having a light chain (LC) and a heavy chain (HC). Antibody light chains can be classified into κ (kappa) and λ (lambda) light chains. Heavy chains can be classified as µ, δ, γ, α, or ε, and define the antibody's isotypes as IgM, IgD, IgG, IgA, and IgE, respectively. Within the light and heavy chains, the variable and constant regions are connected by a "J" region of approximately 12 or more amino acids, and the heavy chain also contains a "D" region of approximately 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain, CL. The constant domain is not directly involved in the binding of antibody to antigen, but exhibits a variety of effector functions, such as mediating the binding of immunoglobulin with host tissue or factor, including various cells of the immune system (e.g., effector cells) and the first component of the classical complement system (C1q). The VH and VL regions can also be subdivided into regions of high variability called complementarity-determining regions (CDRs), interspersed with more conservative regions called framework regions (FRs). Each V_{H} and V_{L} consists of 3 CDRs and 4 FRs arranged from the amino terminus to the carboxyl terminus in the following order: FR1, CDR1, FR2, CDR2, FR3, CDR3, FR4. The variable regions (VH and VL) of each heavy chain/light chain pair respectively form an antigen-binding site. The assignment of amino acids to regions or domains can follow the definition as described in Kabat, Sequences of Proteins of Immunological Interest (National Institutes of Health, Bethesda, Md. (1987 and 1991)), or Chothia & Lesk (1987) J. Mol. Biol. 196 :901-917; Chothia et al. (1989) Nature 342:878-883.

As used herein, the term "complementarity-determining region" or "CDR" refers to those amino acid residues in a variable region of an antibody that are responsible for antigen binding. The variable regions of the heavy chain and light chain each contain three CDRs, named CDR1, CDR2 and CDR3. The precise boundaries of these CDRs can be defined according to various numbering systems known in the art, such as the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al. al., Dev. Comparat. Immunol. 27:55-77, 2003). For a given antibody, one skilled in the art will readily identify the CDRs defined by each numbering system. Moreover, the correspondence between different numbering systems is well known to those skilled in the art (e.g., see Lefranc et al., Dev. Comparat. Immunol. 27:55-77, 2003).

In the present invention, the CDRs contained in the antibody or antigen-binding fragment thereof of the present invention can be determined according to various numbering systems known in the art. In certain embodiments, the CDRs contained in the antibody or antigen-binding fragment thereof of the present invention are preferably determined by the Kabat, Chothia, or IMGT numbering systems. In certain embodiments, the CDRs contained in the antibody or antigen-binding fragment thereof of the present invention are preferably determined by the IMGT numbering system.

As used herein, the term "framework region" or "FR" residues refers to those amino acid residues in an antibody variable region other than the CDR residues as defined above.

The term "antibody" is not limited to any particular method of producing the antibody. It includes, for example, recombinant antibody, monoclonal antibody, and polyclonal antibody. The antibody may be of different isotypes, for example, IgG (e.g., IgG1, IgG2, IgG3 or IgG4 subtypes), IgA1, IgA2, IgD, IgE or IgM antibody.

As used herein, the term "antigen-binding fragment" of an antibody refers to a polypeptide comprising a fragment of a full-length antibody that retains the ability to specifically bind to the same antigen to which the full-length antibody binds, and/or competes with the full-length antibody for specifically binding to the antigen, which is also called an "antigen-binding moiety." See generally, Fundamental Immunology, Ch. 7 (Paul, W., ed., 2nd ed., Raven Press, N.Y. (1989), which is incorporated herein by reference in its entirety for all purposes. Antigen-binding fragments of an antibody can be obtained by recombinant DNA technology or by enzymatic or chemical cleavage of an intact antibody. Non-limiting examples of antigen-binding fragments include Fab, Fab', F(ab')₂, Fd, Fv, complementarity-determining region (CDR) fragment, scFv, diabody, single domain antibody, chimeric antibody, linear antibody, nanobody (technology from Domantis), probody, and such polypeptides, which contain at least a portion of an antibody that is sufficient to confer specificity to the polypeptides with antigen-binding capability. Engineered antibody variants are reviewed in Holliger et al., 2005; Nat Biotechnol, 23: 1126-1136.

As used herein, the term "full-length antibody" refers to an antibody consisting of two "full-length heavy chains" and two "full-length light chains." Among them, "full-length heavy chain" refers to a polypeptide chain that consists of a heavy chain variable region (VH), a heavy chain constant region CH1 domain, a hinge region (HR), a heavy chain constant region CH2 domain, a heavy chain constant region CH3 domain in the direction from the N-terminal to the C-terminal; and, when the full-length antibody is of IgE isotype, it optionally also comprises a heavy chain constant region CH4 domain. Preferably, a "full-length heavy chain" is a polypeptide chain consisting of VH, CH1, HR, CH2 and CH3 in the direction from N-terminal to C-terminal. A "full-length light chain" is a polypeptide chain consisting of a light chain variable region (VL) and a light chain constant region (CL) in the direction from N-terminal to C-terminal. The two pairs of full-length antibody chains are linked together by disulfide bonds between CL and CH1 and between the HRs of the two full-length heavy chains. The full-length antibody of the present invention can be from a single species, such as human; it can also be a chimeric antibody or a humanized antibody. The full-length antibody of the present invention contains two antigen-binding sites formed by VH and VL pairs respectively, and these two antigen-binding sites specifically recognize/bind the same antigen.

As used herein, the term "Fd" refers to an antibody fragment consisting of VH and CH1 domains; the term "dAb fragment" refers to an antibody fragment consisting of a VH domain (Ward et al., Nature 341:544 546 (1989)); the term "Fab fragment" refers to an antibody fragment consisting of VL, VH, CL and CH1 domains; the term "F(ab')₂ fragment" refers to an antibody fragment consisting of two Fab fragments connected by disulfide bridges on the hinge region; the term "Fab' fragment" refers to a fragment obtained by reducing the disulfide bond connecting the two heavy chain fragments in the F(ab')₂ fragment, and consists of a complete light chain and heavy chain Fd fragment (consisting of VH and CH1 domains).

As used herein, the term "Fv" refers to an antibody fragment consisting of VL and VH domains of a single arm of an antibody. Fv fragments are generally considered to be the smallest antibody fragments that can form a complete antigen-binding site. It is generally believed that six CDRs confer the antigen-binding specificity of an antibody. However, even a variable region (e.g., an Fd fragment, which contains only three antigen-specific CDRs) can recognize and bind the antigen, although its affinity may be lower than that of the intact binding site.

As used herein, the term "Fc" refers to an antibody fragment formed by bonding of the second and third constant regions of a first heavy chain of an antibody to the second and third constant regions of a second heavy chain of an antibody via disulfide bonds. The Fc fragment of an antibody has many different functions but does not participate in antigen binding.

As used herein, the term "scFv" refers to a single polypeptide chain comprising VL and VH domains, in which the VL and VH are connected via a linker (see, for example, Bird et al., Science 242:423 -426 (1988); Huston et al., Proc. Natl. Acad. Sci. USA 85:5879-5883 (1988); and Pluckthun, The Pharmacology of Monoclonal Antibodies, Vol. 113, Roseburg and Moore, eds., Springer-Verlag, New York, pp. 269-315 (1994)). Such scFv molecules may have the general structure: NH₂-VL-linker-VH-COOH or NH₂-VH-linker-VL-COOH. Suitable prior art linkers consist of repeated GGGGS amino acid sequences or variants thereof. For example, a linker having the amino acid sequence (GGGGS)₄ can be used, but variants thereof can also be used (Holliger et al. (1993), Proc. Natl. Acad. Sci. USA 90: 6444-6448). Other linkers useful in the present invention are described in Alfthan et al. (1995), Protein Eng. 8:725-731, Choi et al. (2001), Eur. J. Immunol. 31:94-106, Hu et al. (1996), Cancer Res. 56:3055-3061, Kipriyanov et al. (1999), J. Mol. Biol. 293:41-56 and Roovers et al. (2001), Cancer Immunol. In some cases, a disulfide bond may also exist between VH and VL of scFv. In certain embodiments of the present invention, scFv can form di-scFv, which refers to an antibody formed by connecting two or more individual scFvs in series. In certain embodiments of the present invention, scFv can form (scFv)₂, which refers to an antibody formed by connecting two or more individual scFvs in parallel.

As used herein, the term "diabody" refers to an antibody whose VH and VL domains are expressed on a single polypeptide chain, but the linker used is too short to allow pairing between the two domains on the same chain, which forces the domains to pair with the complementary domains of the other chains and generate two antigen-binding sites (see, for example, Holliger P. et al., Proc. Natl. Acad. Sci. USA 90:6444-6448 (1993), and Poljak R. J. et al., Structure 2:1121-1123 (1994)).

As used herein, the term "single-domain antibody (sdAb)" has the common meaning as generally understood by those skilled in the art, and refers to an antibody fragment consisting of a single monomeric variable antibody domain (e.g., a single heavy chain variable region), which retains the ability to specifically bind to the same antigen to which the full-length antibody binds. The single domain antibody is also called nanobody.

Each of the above antibody fragments retains the ability to specifically bind to the same antigen to which the full-length antibody binds, and/or competes with the full-length antibody for specific binding to the antigen.

Antigen-binding fragments of antibody (e.g., the above-described antibody fragments) can be obtained from a given antibody (e.g., the antibody provided by the present invention) using conventional techniques known to those skilled in the art (e.g., recombinant DNA technology or enzymatic or chemical fragmentation methods), and the antigen-binding fragments of the antibody are screened for specificity in the same manner as for intact antibodies.

As used herein, when the term "antibody" is mentioned, it includes not only an intact antibody but also an antigen-binding fragment of the antibody, unless the context clearly indicates otherwise.

As used herein, the term "chimeric antibody" refers to an antibody in which part of the light chain and/or heavy chain is derived from an antibody (which may be derived from a specific species or belong to a specific antibody class or subclass), and the other part of the light chain or/and heavy chain is derived from another antibody (which may be derived from the same or different species or belong to the same or different antibody class or subclass), nevertheless, it still retains the binding activity to the target antigen (U.S.P 4,816,567 to Cabilly et al.; Morrison et al., Proc. Natl. Acad. Sci. USA, 81:6851 6855 (1984)). In certain embodiments, the term "chimeric antibody" may include such an antibody in which the heavy and light chain variable regions of the antibody are derived from a first antibody and the heavy and light chain constant regions of the antibody are derived from a second antibody.

As used herein, the term "identity" is used to refer to the match of sequences between two polypeptides or between two nucleic acids. To determine the percent identity of two amino acid sequences or two nucleic acid sequences, the sequences are aligned for optimal comparison purposes (e.g., gaps may be introduced in the first amino acid sequence or nucleic acid sequence for best alignment with the second amino acid or nucleic acid sequence). The amino acid residues or nucleotides at the corresponding amino acid positions or nucleotide positions are then compared. Molecules are identical when a position in the first sequence is occupied by the same amino acid residue or nucleotide as the corresponding position in the second sequence. The percent identity between two sequences is a function of the number of identical positions shared by the sequences (i.e., percent identity = number of identical overlapping positions/total number of positions × 100%). In certain embodiments, both sequences are of the same length.

Determination of percent identity between two sequences can also be accomplished using mathematical algorithms. One non-limiting example of mathematical algorithm for comparison of two sequences is the algorithm of Karlin and Altschul, 1990, Proc. Natl. Acad. Sci. U.S.A. 87:2264-2268, which was improved by Karlin and Altschul, 1993, Proc. Natl. Acad. Sci. U.S.A. 90:5873-5877. Such algorithms were integrated into the NBLAST and XBLAST programs of Altschul et al., 1990, J. Mol. Biol. 215:403.

As used herein, the term "variant", in the context of polypeptides (including polypeptides), also refers to a polypeptide or peptide comprising an amino acid sequence that has been altered by introducing a substitution, deletion, or addition of amino acid residues. In some cases, the term "variant" also refers to a polypeptide or peptide that has been modified (i.e., by covalently linking any type of molecule to the polypeptide or peptide). For example, and without limitation, polypeptides may be modified, for example, by glycosylation, acetylation, PEGylation, phosphorylation, amidation, derivatization by known protecting/blocking groups, proteolytic cleavage, attachment to cellular ligand or other proteins, etc. Derivatized polypeptides or peptides can be produced by chemical modification using techniques known to those skilled in the art, including, but not limited to, specific chemical cleavage, acetylation, formylation, metabolic synthesis of tunicamycin, and the like. Furthermore, a variant has a similar, identical or improved function to the polypeptide or peptide from which it is derived.

As used herein, the term "specific binding" refers to a non-random binding reaction between two molecules, such as the reaction between an antibody and the antigen it targets. The strength or affinity of a specific binding interaction can be expressed by the equilibrium dissociation constant (K_{D}) of the interaction. In the present invention, the term "K_{D}" refers to the dissociation equilibrium constant of a specific antibody-antigen interaction, which is used to describe the binding affinity between an antibody and an antigen. The smaller the equilibrium dissociation constant, the tighter the antibody-antigen binding, and the higher the affinity between the antibody and the antigen.

The specific binding properties between two molecules can be determined using methods known in the art. One approach involves measuring the rate at which antigen binding site/antigen complex forms and dissociates. Both the "association rate constant" (kₐ or kₒₙ) and the "dissociation rate constant" (k_{dis} or k_{off}) can be calculated from concentrations and actual rates of association and dissociation (see Malmqvist M, Nature, 1993, 361 :186-187). The ratio k_{dis}/kₒₙ is equal to the dissociation constant K_{D} (see Davies et al., Annual Rev Biochem, 1990; 59:439-473). K_{D}, kₒₙ and k_{dis} values can be measured by any valid method. In certain embodiments, dissociation constants can be measured by surface plasmon resonance (SPR) with a Biacore instrument. Besides, bioluminescence interferometry or Kinexa can be used to measure dissociation constants.

As used herein, a detectable label of the present invention may be any substance detectable by fluorescent, spectroscopic, photochemical, biochemical, immunological, electrical, optical or chemical means. Such labels are well known in the art and examples include, but are not limited to, enzymes (e.g., horseradish peroxidase, alkaline phosphatase, beta-galactosidase, urease, glucose oxidase, etc.), radionuclides (e.g., ³H, ¹²⁵I, ³⁵S, ¹⁴C, or ³²P), fluorescent dyes (e.g., fluorescein isothiocyanate (FITC), fluorescein, tetramethylrhodamine isothiocyanate (TRITC), phycoerythrin (PE), Texas red, rhodamine, quantum dots or cyanine dye derivatives (e.g., Cy7, Alexa 750)), luminescent substances (e.g., chemiluminescent substances, such as acridinium esters, luminol and its derivatives compounds, ruthenium derivatives such as ruthenium terpyridyl), magnetic beads (e.g., Dynabeads^{®}), calorimetric markers such as colloidal gold or colored glass or plastic (e.g., polystyrene, polypropylene, latex, etc.) beads, and biotin for binding to avidin (e.g., streptavidin) modified with the above label.

As used herein, the term "vector" refers to a nucleic acid delivery vehicle into which a polynucleotide can be inserted. When the vector can express the protein encoded by the inserted polynucleotide, the vector is called an expression vector. The vector can be introduced into a host cell through transformation, transduction or transfection, so that the genetic material elements it carries can be expressed in the host cell. Vectors are well known to those skilled in the art, including but not limited to: plasmid; phagemid; cosmid; artificial chromosome, such as yeast artificial chromosome (YAC), bacterial artificial chromosome (BAC) or P1-derived artificial chromosome (PAC); phage, such as λ phage or M13 phage and animal virus, etc. Animal viruses that can be used as vectors include, but are not limited to, retrovirus (including lentivirus), adenovirus, adeno-associated virus, herpesvirus (e.g., herpes simplex virus), poxvirus, baculovirus, papillomavirus, papovavirus (e.g., SV40). A vector can contain a variety of expression-controlling elements, including, but not limited to, promoter sequence, transcription initiation sequence, enhancer sequence, selection element, and reporter gene. In addition, the vector may also contain an origin of replication.

As used herein, the term "host cell" refers to a cell that can be used to introduce a vector, which includes, but is not limited to, prokaryotic cell such as *E. coli* or *Bacillus subtilis*, fungal cell such as yeast cell or *Aspergillus*, insect cell such as *S2 Drosophila* cell or Sf9, or animal cell such as fibroblast, CHO cell, COS cell, NSO cell, HeLa cell, BHK cell, HEK 293 cell or human cell.

As used herein, the term "conservative substitution" refers to an amino acid substitution that does not adversely affect or alter the expected properties of the protein/polypeptide comprising the amino acid sequence. For example, conservative substitutions can be introduced by standard techniques known in the art, such as site-directed mutagenesis and PCR-mediated mutation. Conservative amino acid substitutions include those in which an amino acid residue is replaced with an amino acid residue having a similar side chain, for example, one that is physically or functionally similar to the corresponding amino acid residue (e.g., having similar size, shape, charge, chemical properties, including ability to form covalent bonds or hydrogen bonds, etc.). Families of amino acid residues with similar side chains have been defined in the art. These families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine) and aromatic side chains (e.g., tyrosine, phenylalanine, tryptophan, histidine). Therefore, it is preferred to replace the corresponding amino acid residue with another amino acid residue from the same side chain family. Methods for identifying conservative substitutions of amino acids are well known in the art (see, for example, Brummell et al., Biochem. 32:1180-1187 (1993); Kobayashi et al., Protein Eng. 12(10):879-884 (1999); and Burks et al. Proc. Natl Acad. Set USA 94:412-417 (1997), which is incorporated herein by reference).

The twenty conventional amino acids involved herein have been written in accordance with conventional usage. See, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. And in the present invention, amino acids are generally represented by one-letter and three-letter abbreviations well known in the art. For example, alanine can be represented by A or Ala.

As used herein, the term "pharmaceutically acceptable carrier and/or excipient" refers to a carrier and/or excipient that is pharmacologically and/or physiologically compatible with a subject and an active ingredient, and they are well known in the art (see, for example, Remington's Pharmaceutical Sciences. Edited by Gennaro AR, 19th ed. Pennsylvania: Mack Publishing Company, 1995), including, being not limited to: pH adjusting agent, surfactant, adjuvant, ionic strength enhancer, diluent, agent for maintaining osmotic pressure, agent for delaying absorption, preservative. For example, the pH adjusting agent includes, but is not limited to, phosphate buffer. The surfactant includes, but is not limited to, cationic, anionic or nonionic surfactant, such as Tween-80. The ionic strength enhancer includes, but is not limited to, sodium chloride. The preservative includes, but is not limited to, various antibacterial and antifungal agents, such as paraben, chlorobutanol, phenol, sorbic acid, etc. The agent for maintaining osmotic pressure includes, but is not limited to, sugar, NaCl, and the like. The agent for delaying absorption includes, but is not limited to, monostearate and gelatin. The diluent includes, but is not limited to, water, aqueous buffer (e.g., buffered saline), alcohol and polyol (e.g., glycerol), and the like. The preservative includes, but is not limited to, various antibacterial and antifungal agents, such as thimerosal, 2-phenoxyethanol, paraben, chlorobutanol, phenol, sorbic acid, etc. Stabilizers have the meaning generally understood by those skilled in the art, which can stabilize a desired activity of an active ingredient in medicines, including but not limited to sodium glutamate, gelatin, SPGA, saccharide (e.g., sorbitol, mannitol, starch, sucrose, lactose, dextran, or glucose), amino acid (e.g., glutamic acid, glycine), protein (e.g., dry whey, albumin or casein) or degradation product thereof (e.g., lactalbumin hydrolyzate), etc. In certain exemplary embodiments, the pharmaceutically acceptable carrier or excipient includes sterile injectable liquid (e.g., aqueous or non-aqueous suspensions or solutions). In certain exemplary embodiments, such sterile injectable liquid is selected from the group consisting of water for injection (WFI), bacteriostatic water for injection (BWFI), sodium chloride solution (e.g., 0.9% (w/v) NaCl), dextrose solution (e.g., 5% glucose), surfactant-containing solution (e.g., a solution containing 0.01% polysorbate 20), pH buffer solution (e.g., phosphate buffer solution), Ringer's solution and any combination thereof.

As used herein, the term "prevention" refers to a method performed to prevent or delay the occurrence of a disease or condition or symptom in a subject. As used herein, the term "treatment" refers to a method performed to obtain a beneficial or desired clinical outcome. For the purposes of this invention, beneficial or desired clinical outcomes include, but are not limited to, alleviation of symptoms, reduction of the extent of disease, stabilization (i.e., no worsening) of the state of disease, delaying or slowing the progression of disease, ameliorating or alleviating the status of disease, and relief of symptoms (whether partial or complete), whether detectable or undetectable. In addition, "treatment" may also refer to prolonging survival compared to expected survival if not receiving treatment.

As used herein, the term "subject" refers to a mammal, such as a human, a cynomolgus monkey, a mouse. In certain embodiments, the subject (e.g., human, cynomolgus monkey, mouse) has a disease associated with TIGIT (e.g., a tumor involving TIGIT-positive infiltrating T cell and/or NK cell, and/or involving TIGIT ligand (e.g., CD155 and/or CD112)-positive tumor cell), or, is at a risk of suffering from the aforementioned disease.

As used herein, the term "effective amount" refers to an amount sufficient to achieve, or at least partially achieve, the desired effect. For example, an effective amount for preventing a disease (e.g., a tumor involving TIGIT-positive infiltrating T cell and/or NK cell, and/or involving TIGIT ligand (e.g., CD155 and/or CD112)-positive tumor cell) refers to an amount that is sufficient to prevent, arrest or delay the occurrence of the disease; the effective amount for treating a disease refers to an amount that is sufficient to cure or at least partially prevent the disease and its complications in a patient who already has the disease. Determining such effective amounts is well within the capabilities of those skilled in the art. For example, the amount effective for therapeutic use will depend on the severity of the disease to be treated, the overall status of the patient's own immune system, the patient's general condition such as age, weight and gender, the manner in which the drug is administered, and other treatments administered concurrently, and so on.

### Beneficial effects of the present invention

The present invention provides fully human antibodies against TIGIT, which have high binding affinity to TIGIT, can effectively block the binding of TIGIT to its ligand CD155/CD112, thereby reducing or eliminating the inhibitory signal transmitted to cells, and the administration of the antibody of the present invention in animal models can significantly inhibit tumor growth. Therefore, the antibodies of the present invention can be used for a variety of purposes, including but not limited to enhancing immune responses, inhibiting tumor growth, anti-infection and detecting TIGIT. Furthermore, the fully human antibodies of the present invention can be safely administered to human subjects without eliciting an immunogenic response. Therefore, the antibodies of the present invention have significant clinical value.

The embodiments of the present invention will be described in detail below with reference to the accompanying drawings and examples, but those skilled in the art will understand that the following drawings and examples are only used to illustrate the present invention and do not limit the scope of the present invention. Various objects and advantageous aspects of the present invention will become apparent to those skilled in the art from the accompanying drawings and the following detailed description of preferred embodiments.

### Brief Description of the Drawings

Fig. 1 shows the binding activity of the anti-TIGIT antibody of the present invention to human TIGIT overexpressed on CHO cells.
Fig. 2 shows the binding activity of the anti-TIGIT antibody of the present invention to cynomolgus TIGIT overexpressed on CHO cells.
Fig. 3 shows the binding activity of the anti-TIGIT antibody of the present invention to mouse TIGIT overexpressed on CHO cells.
Fig. 4 shows the blocking activity of the anti-TIGIT antibody of the present invention in blocking the binding of human TIGIT overexpressed on CHO cells to human CD155.
Fig. 5 shows the blocking activity of the anti-TIGIT antibody of the present invention in blocking the binding of mouse TIGIT overexpressed on CHO cells to mouse CD155.
Fig. 6 shows the binding activity of the anti-TIGIT antibody of the present invention to TIGIT on activated human primary T cells.
Fig. 7 shows the blocking activity of the anti-TIGIT monoclonal antibody of the present invention in blocking the TIGIT/CD155 signaling.
Figs. 8A to 8B show the blocking activity of the anti-TIGIT antibody of the present invention and anti-PD-L1/PD-1 molecule in synergistically blocking TIGIT/CD155/CD112 and PD-L1/PD-1 signaling.
Figs. 9A to 9C show the in vitro ADCC activity of the anti-TIGIT antibody of the present invention.
Figs. 10A to 10B show the drug efficacy of the anti-TIGIT antibody of the present invention in the CT-26 tumor-bearing wild-type mouse model.
Fig. 11 shows the synergistic drug efficacy of the anti-TIGIT antibody of the present invention and anti-PD-L1 antibody in the CT-26 tumor-bearing wild-type mouse model.
Figs. 12A to 12B shows the synergistic drug efficacy of the anti-TIGIT antibody of the present invention and anti-PD-L1 antibody in the CT-26 tumor-bearing huTIGIT KI mouse model.
Fig. 13 shows the synergistic drug efficacy of the anti-TIGIT antibody of the present invention and anti-PD-L1 antibody in the B-NDG mouse model inoculated with mixed A375 and human PBMC.
Figs. 14A to 14B show the half-life of the anti-TIGIT antibody of the present invention in mice.

### Sequence Information

A description of the sequences covered by the present application is provided in the table below.

**Table 1: Sequence information**

| SEQ ID NO: | Sequence and description |
|---|---|
| 1 | ADI-55796 VH CDR1 |
| | GGSISSYDHY |
| 2 | ADI-55796 VH CDR2 |
| | VYYSGST |
| 3 | ADI-55796 VH CDR3 |
| | ARVGPDVSHPPFDY |
| 4 | ADI-55796 VH |
| | QLQLQESGPGLVKPAETLSLTCTVSGGSISSYDHYWTWIRQPPGKGLEWIGTVY YSGSTFHNPSLKSRVTIPVDTSKNQFSLKLSSVTAADTAVYYCARVGPDVSHPPF DYWGQGTLVTVSS |
| 5 | ADI-55796 VL CDR1 |
| | QSISSY |
| 6 | ADI-55796 VL CDR2 |
| | AAS |
| 7 | ADI-55796 VL CDR3 |
| | QQSYSTPIT |
| 8 | ADI-55796 VL |
| | DIQMTQSPSSLSASVGDRVTITCRASQSISSYLNWYQQKPGKAPKLLIYAASSLQ SGVPSRFSGSGSGTDFTLTISSLQPEDFATYYCQQSYSTPITFGGGTKVEIK |
| 9 | ADI-55812 VH CDR1 |
| | GYAFTGYY |
| 10 | ADI-55812 VH CDR2 |
| | IIPFSGEA |
| 11 | ADI-55812 VH CDR3 |
| | ARGPGSLDRLW YYYYGMDV |
| 12 | ADI-55812 VH |
| | QVQLVQSGAEVKKPGASVKVSCKASGYAFTGYYMHWVRQAPGQGLEWMGSII PFSGEANYAQKFQGRVTMTRDTSISTAYMELSRLRSDDTAVYYCARGPGSLDRL WYYYYGMDVWGQGTTVTVSS |
| 13 | ADI-55812 VL CDR1 |
| | QSVSSSY |
| 14 | ADI-55812 VL CDR2 |
| | GAS |
| 15 | ADI-55812 VL CDR3 |
| | QQYGSSPIT |
| 16 | ADI-55812 VL |
| | EIVLTQSPGTLSLSPGERATLSCRASQSVSSSYLAWYQQKPGQAPRLLIYGASSR ATGIPDRFSGSGSGTDFTLTISRLEPEDFAVYYCQQYGSSPITFGGGTKVEIK |
| 17 | Amino acid sequence of heavy chain constant region of wild-type human IgG1 |
| | ASTKGPSVFPLAPSSKSTSGGTAALGCLVKDYFPEPVTVSWNSGALTSGVHTFPA VLQSSGLYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALPAPIE KTISKAKGQPREPQVYTLPPSRDELTKNQVSLTCLVKGFYPSDIAVEWESNGQPE NNYKTTPPVLDSDGSFFLYSKLTVDKSRWQQGNVFSCSVMHEALHNHYTQKSL SLSPG |
| 18 | Amino acid sequence of heavy chain constant region of IgG1 with mutation L234A and L235A (G1LALA) |
| | ASTKGPSVFPLAPSSKSTSGGTAALGCLVKDYFPEPVTVSWNSGALTSGVHTFPA VLQSSGLYSLSSVVTVPSSSLGTQTYICNVNHKPSNTKVDKKVEPKSCDKTHTC PPCPAPEAAGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALPAPIE KTISKAKGQPREPQVYTLPPSRDELTKNQVSLTCLVKGFYPSDIAVEWESNGQPE NNYKTTPPVLDSDGSFFLYSKLTVDKSRWQQGNVFSCSVMHEALHNHYTQKSL SLSPG |
| 19 | Amino acid sequence of human TIGIT extracellular region |
| | MMTGTIETTGNISAEKGGSIILQCHLSSTTAQVTQVNWEQQDQLLAICNADLGW HISPSFKDRVAPGPGLGLTLQSLTVNDTGEYFCIYHTYPDGTYTGRIFLEVLESSV AEHGARFQIP |
| 20 | Amino acid sequence of cynomolgus TIGIT extracellular region |
| | KPGFSETVFSHRLSFTVLSAVGYFRWQKRPHLLPVSPLGRSMRWCLFLIWAQGL RQAPLASGMMTGTIETTGNISAKKGGSVILQCHLSSTMAQVTQVNWEQHDHSL LAIRNAELGWHIYPAFKDRVAPGPGLGLTLQSLTMNDTGEYFCTYHTYPDGTYR GRIFLEVLESSVAEHSARFQIP |
| 21 | Amino acid sequence of mouse TIGIT extracellular region |
| | TIDTKRNISAEEGGSVILQCHFSSDTAEVTQVDWKQQDQLLAIYSVDLGWHVAS VFSDRVVPGPSLGLTFQSLTMNDTGEYFCTYHTYPGGIYKGRIFLKVQESSDDR NGLAQFQTAPLG |
| 22 | Amino acid sequence of kappa light chain constant region of human immunoglobulin |
| | |

### Specific Models for Carrying Out the present invention

The present invention will now be described with reference to the following examples which are intended to illustrate but not to limit the present invention.

Unless otherwise specified, the molecular biology experimental methods and immunoassay methods used in the present invention were carried out basically referring to the methods described by J. Sambrook et al., Molecular Cloning: A Laboratory Manual, 2nd Edition, Cold Spring Harbor Laboratory Press, 1989, and F. M. Ausubel et al., Compiled Laboratory Guide to Molecular Biology, 3rd Edition, John Wiley & Sons, Inc., 1995; the use of restriction enzymes was in accordance with the conditions recommended by the product manufacturer. Those skilled in the art will appreciate that the examples describe the present invention by way of example and are not intended to limit the scope of the present invention as claimed.

### Example 1: Preparation of antibody

### 1.1 Screening of fully human anti-TIGIT antibody by yeast display technology

Based on the yeast display antibody library (Adimab), amplification was performed according to the existing methods (see patent applications WO2009036379, WO2010105256 and WO2012009568), in which the diversity of each library reached 1×10⁹. Briefly, in the first two rounds of screening, Miltenyi's MACS system was used for magnetic bead cell sorting. First, the yeast cells in the library (∼1×10¹⁰ cells/library) were incubated in FACS wash buffer (phosphate buffer containing 0.1% BSA) for 15 minutes at room temperature, in which the buffer contained 100 nM biotin-labeled human TIGIT (Purchased from Acro, Cat. No.: TIT-H5254) as the antigen. Washing was performed once with 50 mL of pre-chilled FACS wash buffer, then the cells were suspended in 40 mL of the buffer, 500 µL of streptavidin magnetic beads (Miltenyi LS) was added, and incubated at room temperature for 15 minutes. After centrifugation at 1000 rpm for 5 minutes and discarding the supernatant, the cells were resuspended in 5 mL of FACS wash buffer, and the cell suspension was loaded to the Miltenyi LS column. After the completion of loading the sample, the column was washed three times with FACS wash buffer, 3 mL each time. The Miltenyi LS column was taken out from the magnetic area, eluted with 5 mL of growth medium, and the eluted yeast cells were collected and allowed to grow at 37°C overnight.

Flow cytometry was used for the next round of sorting: Approximately 1×10⁸ yeast cells obtained through MACS system screening were washed three times with FACS buffer, and incubated in a solution containing low concentrations of biotin-labeled TIGIT antigen (1 nM, 10 nM, 100 nM) at room temperature. The culture medium was discarded, and the cells were washed twice with FACS wash buffer. The cells were then mixed with LC-FITC (FITC-labeled anti-human immunoglobulin kappa light chain antibody, purchased from Southern Biotech) (1:100 dilution), and mixed with SA-633 (Streptavidin-633, purchased from Molecular Probes) (1:500 dilution) or SA-PE (Streptavidin-PE, purchased from Sigma) (1:50 dilution), and incubated at 40°C for 15 minutes. Elution was performed twice with pre-chilled FACS wash buffer, and the cells were resuspended in 0.4 mL of the buffer. The cells were transferred to a separation tube with a filter. The cells were sorted using FACS ARIA (BD Biosciences).

The yeast cells obtained through screening were shaken and induced at 30°C for 48 hours to secrete and express the target anti-TIGIT antibody (full-length IgG). After induction, the yeast cells were removed by centrifugation at 1300 rpm for 10 minutes, and the supernatant was collected. Protein A was used to purify the anti-TIGIT antibody in the supernatant, elution was performed with pH 2.0 acetic acid solution, and the anti-TIGIT antibody was harvested.

### 1.2 Optimization of affinity of anti-human TIGIT antibody

In order to obtain higher affinity anti-human TIGIT antibody, the antibody obtained through the above screening was optimized through, but not limited to, the following method.

### 1.2.1 CDRH1/CDRH2 screening

The CDRH3 gene of the parent molecule was constructed into a CDRH1/CDRH2 gene library with a diversity of 1×10⁸, and three rounds of screening were performed. The MACS method was used in the first round, and FACS was used in the second and third rounds. The antibody-antigen conjugate was subjected to affinity stress to screen out a high-affinity antibody.

The yeast cells expressing anti-TIGIT antibody obtained through screening were cultured with shaking at 30°C for 48 hours to produce TIGIT antibody. After the induced expression was completed, the yeast cells were removed by centrifugation at 1300 rpm for 10 minutes, and the supernatant was harvested. Protein A was used to purify the anti-TIGIT antibody in the supernatant, elution was performed with pH 2.0 acetic acid solution, and the anti-TIGIT antibody was harvested. Purification was performed with papain and KappaSelect (GE Life Healthcare) to obtain the corresponding Fab fragment.

### 1.2.2 VHmut screening

In this method, a mutation was introduced into a heavy chain region through conventional error-prone PCR method. During the PCR process, the base mismatch probability was increased to approximately 0.01 bp by using 1 µM highly mutagenic base analogues dPTP and 8-oxo-dGTP.

The product obtained by error-prone PCR was constructed into a vector containing the heavy chain constant region by homologous recombination. Through this method, under the screening stresses including TIGIT antigen titer, unlabeled antigen competition, and competition using parent antibody, a secondary library with a library capacity of 1×10⁷ was obtained, three rounds of screening were performed by FACS method, and the yeast cells expressing corresponding antibodies were obtained.

### 1.2.3 CDRL1/CDRL2/CDRL3 screening

The CDRL3 diversity genes were constructed into the CDRL1/CDRL2 gene library through homologous recombination, and a secondary library with a library capacity of 1×10⁷ was obtained. Screening was performed by one round of MACS and two rounds of FACS, and the yeast cells expressing corresponding antibodies were obtained.

In this example, affinity matured antibody molecules ADI-55796 and ADI-55812 were obtained. The sequences and sequence numbers of ADI-55796 and ADI-55812 were shown in Table 1, wherein the CDRs were determined by the IMGT numbering system.

### 1.3 Expression and purification of antibodies

The following control antibody used in the example was expressed and purified from HEK293 cells: Tiragolumab, which was an anti-human TIGIT antibody expressed by HEK293 cells from Genentech, and its light and heavy chain variable region sequences were consistent with those of INN list 177 NO 1918185-84-8.

For candidate clones ADI-55796 and ADI-55812, their heavy chain variable regions were constructed to the heavy chain constant region of wild-type human IgG1 (the amino acid sequence was set forth in SEQ ID NO: 17), and the heavy chain constant region of human IgG1 with mutation L234A and L235A (G1LALA) (the amino acid sequence was set forth in SEQ ID NO: 18), respectively; and their light chain variable regions were constructed to the human immunoglobulin kappa light chain constant region (the amino acid sequence was set forth in SEQ ID NO: 22). The antibody was transiently expressed through the HEK293 expression system and purified, and the specific operation was as follows: the pcDNA3.1 vector with heavy and light chains of antibody was transferred into HEK293 cells by chemical transfection method, cultured at 37°C, 8% CO₂ for 7 days. The cell fluid was collected and centrifuged at 13,000 rpm for 20 minutes. The supernatant was taken, purified with Protein A, and the antibody purity was detected by SEC, while the endotoxin content was controlled. Finally, antibodies ADI-55796-G1, ADI-55812-G1, ADI-55796-G1LALA, and ADI-55812-G1LALA were obtained.

### Example 2: Detection of antibody affinity

Biofilm layer optical interference technology (ForteBio) was used to determine the binding and dissociation rate constant (K_{D}) values of the antibodies ADI-55796-G1, ADI-55812-G1, ADI-55796-G1LALA, and ADI-55812-G1LALA obtained in Example 1 to TIGITs derived from human, cynomolgus monkey, and mouse. Fortebio affinity measurement was performed according to existing methods (Este, P et al., High throughput solution-based measurement of antibody-antigen affinity and epitope binning. Mabs, 2013.5(2):p.270-8), the amino acid sequences of the extracellular region of the TIGITs of human, cynomolgus monkey and mouse were set forth in SEQ ID NO: 19-21, respectively.

The monovalent affinities of intact IgG antibodies to human, cynomolgus, and mouse TIGIT-his proteins were measured, in which a sensor was equilibrated offline in an analysis buffer for 20 minutes, then online detection was carried out for 120 s to establish a baseline, and the intact TIGIT antibodies were loaded onto AHQ sensor to reach a thickness of 1 nm for affinity detection. The antibody-loaded sensor was incubated in 100 nM TIGIT-his antigen to the plateau phase, and then the sensor was transferred to an analysis buffer and incubated for at least 2 minutes for measuring dissociation rate. The kinetic analysis was performed using a 1:1 binding model.

In the above determination method, the K_{D} values of the molecules were shown in Table 2:

**Table 2: K_{D} values of anti-TIGIT antibodies**

| Antibody | Monovalent affinity of IgG to human TIGIT-his protein | Monovalent affinity of IgG to cynomolgus TIGIT-his protein | Monovalent affinity of IgG to mouse TIGIT-his protein |
|---|---|---|---|
| ADI-55796-G1 | 1.99E-10 | 4.72E-09 | 9.60E-08 |
| ADI-55796-G1LALA | 2.10E-10 | 4.90E-09 | 6.10E-08 |
| ADI-55812-G1 | 3.50E-09 | 2.41E-08 | N.B. |
| ADI-55812-G1LALA | 3.40E-09 | 2.21E-08 | N.B. |
| Tiragolumab | 8.70E-10 | 3.50E-09 | NA |

| | | | |
|---|---|---|---|
| Note: "N.B.": no binding; "NA": not available. | | | |

It could be seen from the results in Table 2 that: (1) both anti-TIGIT clones ADI-55796 and ADI-55812 had high binding activity to human TIGIT and cynomolgus TIGIT, and in addition, ADI-55796 also had cross-binding activity to mouse TIGIT.

### Example 3: Binding activity and blocking activity of anti-TIGIT antibodies to human/cynomolgus/mouse TIGIT-overexpressing CHO cells

### 3.1 Detection of binding activity of anti-TIGIT antibodies to human/cynomolgus/mouse TIGIT overexpressed on CHO cells based on flow cytometry

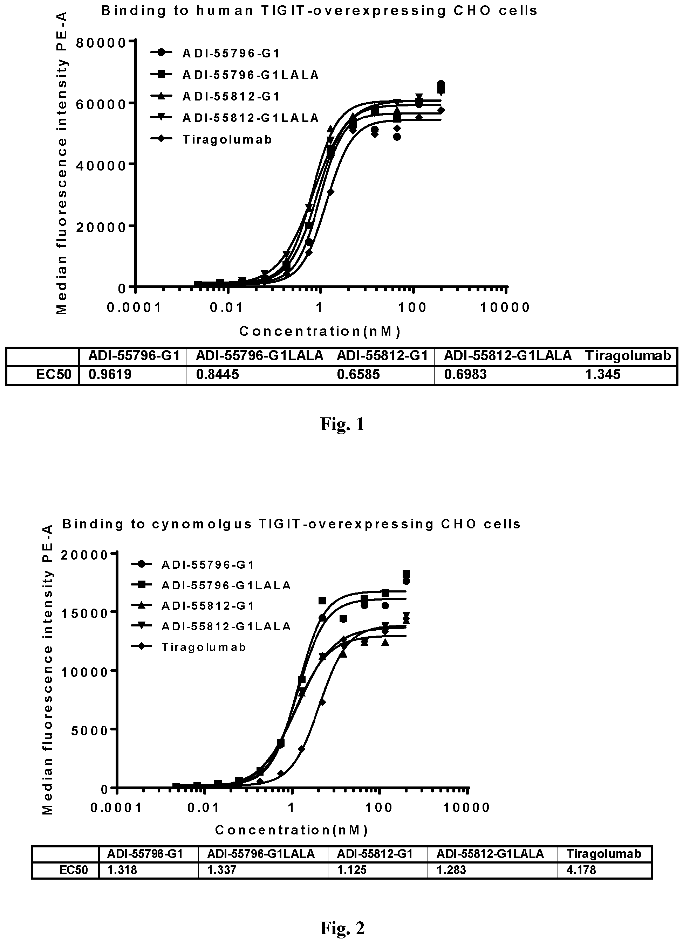

Specifically, human TIGIT-overexpressing CHO-S cells (CHO-huTIGIT cells), cynomolgus TIGIT-overexpressing CHO-S cells (CHO-cynoTIGIT cells), and mouse TIGIT-overexpressing CHO-S cells (CHO-muTIGIT cells) were generated by transfection of the pCHO1.0 vector (purchased from Invitrogen) with cDNA encoding human TIGIT, cynomolgus TIGIT, and mouse TIGIT cloned into multiple cloning site (MCS), respectively, and by the subsequent pressure screening. The overexpressing cells were expanded and adjusted to a suitable cell density, and added to a 96-well flow cytometry plate. After centrifugation, the cells were added with a gradiently diluted sample to be tested, and incubated at 4°C for 30 minutes. The cells were washed twice with PBS, and added with a fluorescent secondary antibody correspondingly diluted to an appropriate concentration, then incubated at 4°C for 30 minutes, and washed twice with PBS. The cells were resuspended with PBS, and detected on a CytoFlex flow cytometer, and the corresponding MFI was calculated. Graphpad software was used for graphing analysis to obtain EC50 values. The results were shown in Table 3 and Figs. 1 to 3.

### 3.2 Flow cytometry detection of blocking activity of anti-TIGIT antibodies in blocking the binding of human TIGIT overexpressed on CHO cells to human CD155, and in blocking the binding of mouse TIGIT overexpressed on CHO cells to mouse CD155

Specifically, the expanded CHO-huTIGIT cells were adjusted to a cell density of 2×10⁶ cells/mL, added at 100 µL/well to a 96-well flow plate, and centrifuged for later use. The purified monoclonal antibody was 3-folds diluted with PBS starting with 400 nM for a total of 12 concentration gradients. The diluted sample was added at 60 µL/well to the cells-carrying 96-well flow plate, and incubated at 4°C for 30 minutes. Then, human CD155 protein with mouse IgG2a Fc Tag was added at 60 µL/well to reach a final concentration of 2 µg/mL, incubated at 4°C for 30 minutes, and washed twice with PBS. APC-labeled goat anti-mouse IgG antibody diluted 100-folds with PBS was added at 100 µL/well, incubated at 4°C for 30 minutes, and washed twice with PBS. PBS was added at 100 µL/well to resuspend the cells, and the detection was performed on the CytoFlex flow cytometer and the corresponding MFI was calculated.

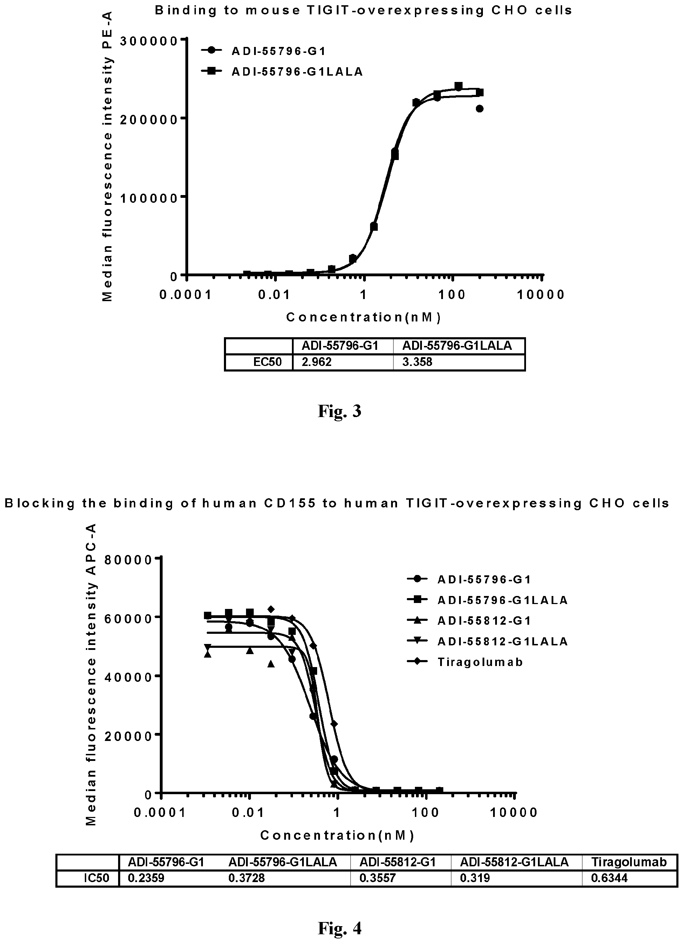

The expanded CHO-muTIGIT cells were adjusted to have a cell density of 2×10⁶ cells/mL, added at 100 µL/well to a 96-well flow plate, and centrifuged for later use. The purified monoclonal antibody was 3-folds diluted with PBS starting with 400 nM for a total of 12 concentration gradients. The diluted sample was added at 60 µL/well to the cells-carrying flow plate, and incubated at 4°C for 30 minutes. Then, mouse CD155 protein with mouse IgG2a Fc Tag was added at 60 µL/well to reach a final concentration of 2 µg/mL, incubated at 4°C for 30 minutes, and washed twice with PBS. APC-labeled goat anti-mouse IgG antibody diluted 100-folds with PBS was added at 100 µL/well, incubated at 4°C for 30 minutes, and washed twice with PBS. PBS was added at 100 µL/well to resuspend the cells, and the detection was performed on the CytoFlex flow cytometer and the corresponding MFI was calculated. Graphpad software was used for graphing analysis to obtain IC50 values. The results were shown in Table 3 and Figs. 4 to 5.

**Table 3. Summary table of the binding activity and the blocking activity of anti-TIGIT antibodies on overexpressing cells**

| Antibody | EC50 of binding human TIGIT-overexpressin g CHO cells (nM) | EC50 of binding cynomolgus TIGIT-overexpressi ng CHO cells (nM) | EC50 of binding mouse TIGIT-overexpressi ng CHO cells (nM) | IC50 of blocking the binding of human TIGIT-overexpressi ng CHO cells to human CD155 (nM) | IC50 of blocking the binding of mouse TIGIT-overexpressin g CHO cells to mouse CD155 (nM) |
|---|---|---|---|---|---|
| ADI-55796-G1 | 0.96 | 1.32 | 2.96 | 0.24 | 1.50 |
| ADI-55796-G1LALA | 0.84 | 1.34 | 3.36 | 0.37 | 1.56 |
| ADI-55812-G1 | 0.66 | 1.13 | N.B. | 0.36 | NA |
| ADI-55812-G1LALA | 0.70 | 1.28 | N.B. | 0.32 | NA |
| Tiragolumab | 1.35 | 4.18 | N.B. | 0.63 | NA |

| | | | | | |
|---|---|---|---|---|---|
| Note: "N.B.": no binding; and "NA": not available. | | | | | |

The following conclusions could be drawn from Table 3 and Figs. 1 to 3: (1) The binding activities of ADI-55796 and ADI-55812 to human TIGIT and cynomolgus TIGIT were better than those of the control molecule Tiragolumab; and (2) ADI-55796 showed significant binding to mouse TIGIT protein overexpressed on the surface of CHO cells.

The following conclusions could be drawn from Table 3 and Figs. 4 to 5: (1) The blocking activities of ADI-55796 and ADI-55812 in blocking the binding of the overexpressed human TIGIT protein on the surface of CHO cells to human CD155 were better than those of the control molecule Tiragolumab; and (2) The anti-TIGIT antibody molecule ADI-55796, which showed binding to the mouse TIGIT protein overexpressed on the surface of CHO cells, could also significantly block the binding of the mouse TIGIT protein overexpressed on the surface of CHO cells to mouse CD155.

### Example 4: Binding of anti-TIGIT antibody to TIGIT on the surface of primary T cells

The binding activity of the anti-TIGIT antibody of the present invention to TIGIT on the surface of activated T cells was detected based on the flow cytometry detection method.

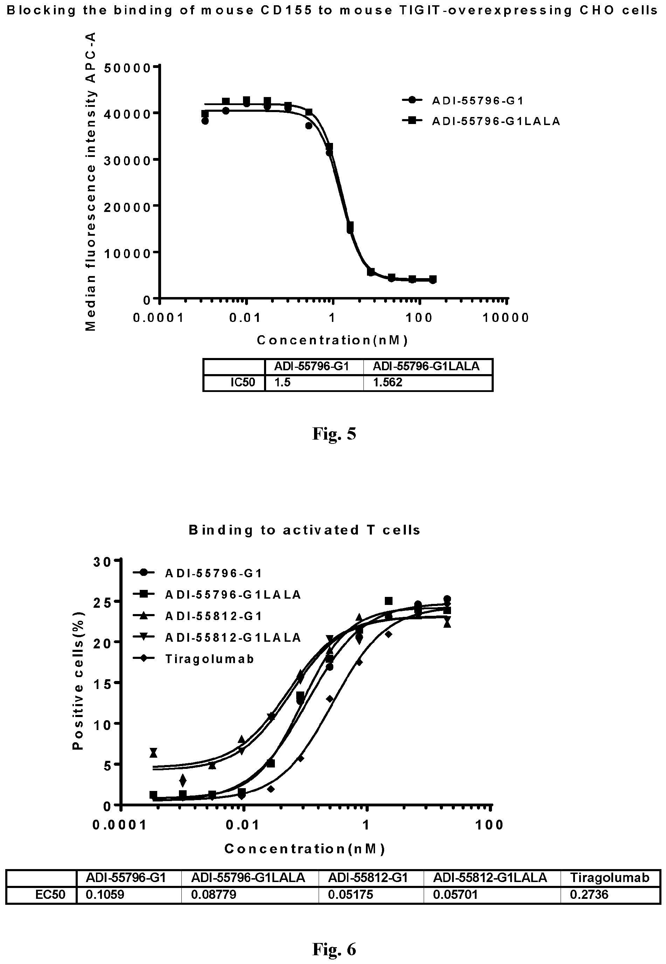

Specifically, human PBMCs were sorted according to the experimental protocol provided by STEMCELL Company (stemcell, Cat. No.: #17951C) to obtain human total T cells. The T cells were adjusted to have a concentration of 1.0×10⁶ cells/mL using X-VIVO15 medium (purchased from lonza, Cat. No.: 04-418Q), added with 1µL of IL-2 stock solution (1,000,000 IU), and added at the same time with CD3/CD28 Dynabeads (purchased from gibco, Cat. No.: 11132D) at 1:1 (bead-to-cell), and cultured in a 37°C, 5% CO₂ incubator for 48 hours. The activated T cells were adjusted to a suitable cell density and added to a 96-well flow cytometry plate. After centrifugation, the gradiently diluted sample to be tested was added, and incubated at 4°C for 30 minutes. Washing was carried out twice with PBS, and a fluorescent secondary antibody correspondingly diluted to an appropriate concentration was added, incubated at 4°C for 30 minutes, and washed twice with PBS. PBS was added to resuspend the cells, and the detection was performed on the CytoFlex flow cytometer and the corresponding MFI was calculated. The results were shown in Fig. 6. The results showed that the anti-TIGIT antibodies ADI-55796-G1, ADI-55796-G1LALA, ADI-55812-G1, and ADI-55812-G1LALA of the present invention could bind to TIGIT molecules on the surface of activated T cells, and had binding activity better than that of the control molecule Tiragolumab.

### Example 5: Detection of anti-TIGIT antibody activity by luciferase reporter gene system

In order to further detect the blocking activity of TIGIT antibodies at the cellular level, a luciferase reporter gene system was constructed in this example. Briefly, lentivirus was used to transfect cells to construct a CHO-K1 cell strain (CHO-K1-CD155) overexpressing human CD155 and OKT-3 scFv, and to construct a Jurkat cell strain (Jurkat-TIGIT-luc) overexpressing human TIGIT and comprising NF-AT luciferase reporter genes, and this reporter gene system was subsequently used to carry out relevant experiments.

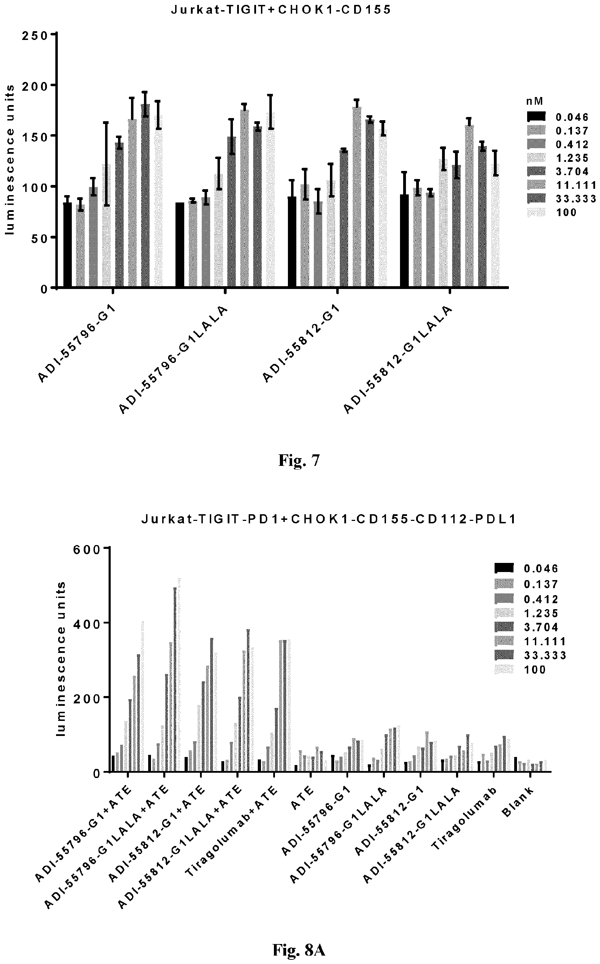

Specifically, CHO-K1-CD155 functional cells were obtained by digestion, adjusted to have a desired cell density, added at 100 µL/well to a 96-well white bottom plate, and underwent adherent culture overnight. On the next day, a Jurkat-TIGIT-luc effector cell suspension was prepared, the sample to be tested was gradiently diluted with reaction medium (RPMI1640+10%FBS). The white bottom plate was taken out, the culture supernatant was discarded by pipetting, the above diluted sample was added at 40 µL/well to the white bottom plate, the Jurkat-TIGIT-luc effector cell suspension was added at 40 µL/well at the same time, and the culturing was carried out in a 37°C, 5% CO₂ incubator for 6 hours. During this period, Bio-Glo TM reagent (purchased from promega, Cat. No.: G7940) was returned to room temperature. After the culturing was completed, the cells were taken out and equilibrated at room temperature for 5 minutes, and added with Bio-Glo TM reagent at 80 µL/well, and a multifunctional microplate reader was used to read luminescence signal values. The results were shown in Fig. 7. The results showed that the anti-TIGIT antibodies ADI-55796-G1, ADI-55796-G1LALA, ADI-55812-G1, and ADI-55812-G1LALA of the present invention demonstrated blocking activity in blocking the CD155-mediated TIGIT downstream signaling, and could upregulate the luciferase expression of reporter gene.

### Example 6: Detection of synergistic effect of anti-TIGIT antibody and anti-PD-L1/PD-1 antibody by luciferase reporter gene system

In order to further detect the synergistic blocking activity of TIGIT antibody and anti-PD-1/PD-L1 antibody at the cellular level, the following luciferase reporter gene system was constructed in this example. Briefly, based on Example 5, lentivirus was used to infect CHO-K1-CD155 to overexpress CD122 and PD-L1 to obtain CHO-K1-CD155-CD112-PD-L1 functional cells, and lentivirus was used infect Jurkat-TIGIT-luc to overexpress PD-1 to obtain Jurkat-TIGIT-PD-1-luc effector cell suspension, and this reporter gene system was used in subsequent experiments.

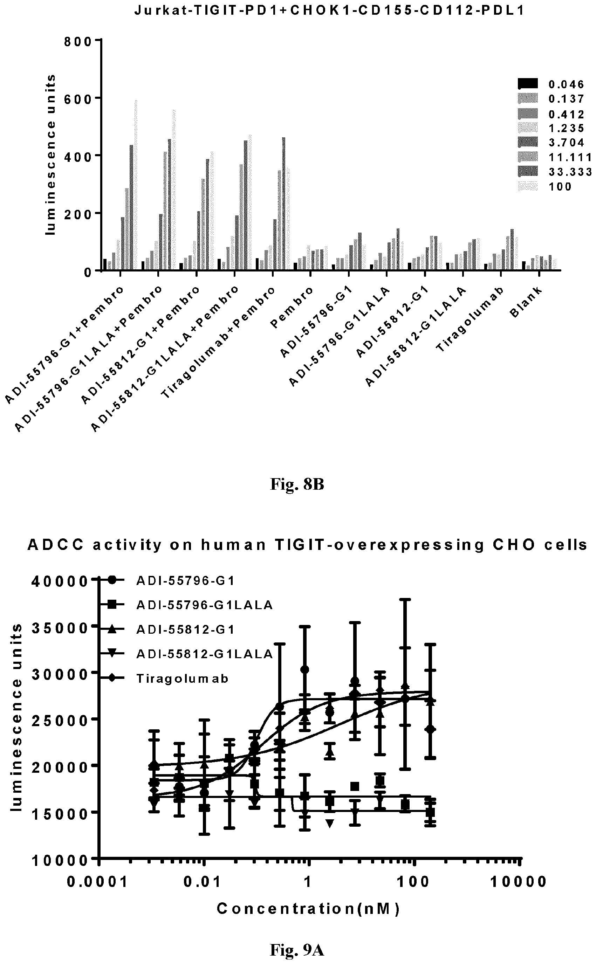

Specifically, CHO-K1-CD155-CD112-PD-L1 functional cells were obtained by digestion, adjusted to have a desired cell density, added at 100 µL/well to a 96-well white bottom plate, and underwent adherent culture overnight. On the next day, a Jurkat-TIGIT-PD-1-luc effector cell suspension was prepared, and the sample to be tested (wherein, the anti-PD-1/PD-L1 antibodies used were Atezolizumab and Pembrolizumab) was gradiently diluted with the reaction medium. The white bottom plate was taken out, the culture supernatant was discarded by pipetting, the above diluted sample was added at 40 µL/well to the white bottom plate, the Jurkat-TIGIT-PD-1-luc effector cell suspension was added at 40 µL/well, and the culturing was carried out in a 37°C, 5% CO₂ incubator for 6 hours. During the period, the Bio-Glo TM reagent was returned to room temperature. After the culturing was completed, the cells were taken out and equilibrated at room temperature for 5 minutes, added with Bio-Glo TM reagent at 80 µL/well, and a multifunctional microplate reader was used to read luminescence signal values. The results were shown in Figs. 8A to 8B. The results showed that the anti-TIGIT antibodies ADI-55796-G1, ADI-55796-G1LALA, ADI-55812-G1, and ADI-55812-G1LALA of the present invention and the anti-PD-1/PD-L1 antibody could synergistically relieve the CD 155-mediated TIGIT downstream inhibitory signaling, and the PD-L1-mediated PD-1 downstream inhibitory signaling, thereby up-regulating the luciferase expression of reporter gene.

### Example 7: In vitro ADCC activity detection of anti-TIGIT antibody

Based on the luciferase reporter gene system, the in vitro ADCC activity of the anti-TIGIT antibodies of the present invention was detected.

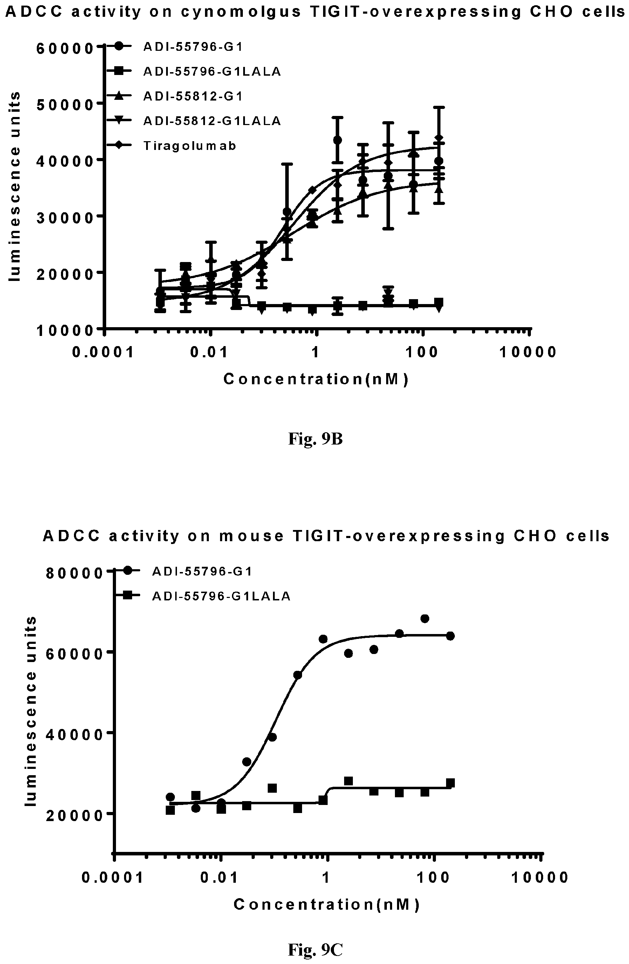

Specifically, Jurkat-NFAT-Luciferase-CD16 ADCC effector cells (purchased from Promega) were expanded, and the cells were resuspended in RPMI1640 medium containing 10% low IgG FBS to 4×10⁶ cells/mL. The CHO-huTIGIT cells, CHO-cynoTIGIT cells, and CHO-muTIGIT cells were resuspended in RPMI1640 medium containing 10% low IgG FBS, and diluted to 1.6×10⁶ cells/mL. The above three cell suspensions were mixed with the Jurkat-NFAT-Luciferase-CD16 cells at a ratio of 1:1, respectively, added at 50 µL/well into a sterile 96-well white bottom plate, and added with the antibody sample to be tested that was gradiently diluted in RPMI1640 medium containing 10% low IgG FBS. Co-incubation was carried out at 37°C, 5% CO₂ for 6 hours. After the culturing was completed, the cells were taken out and equilibrated at room temperature for 5 minutes, added with Bio-Glo TM reagent at 100 µL/well, and a multifunctional microplate reader was used to read luminescence signal values. The results were shown in Figs. 9A to 9C. The results showed that the anti-TIGIT antibodies ADI-55796-G1 and ADI-55812-G1 of the present invention could mediate the ADCC activity on CHO-huTIGIT cells, CHO-cynoTIGIT cells and CHO-muTIGIT cells in vitro by the luciferase reporter gene system.

Example 8: In vivo pharmacodynamic study on anti-TIGIT antibody in wild-type Balb/c mice In this experiment, wild-type Balb/c mice were inoculated with CT-26 colon cancer cells (purchased from Gempharmatech Co., Ltd.) to determine the anti-tumor effect of the anti-TIGIT antibody of the present invention.

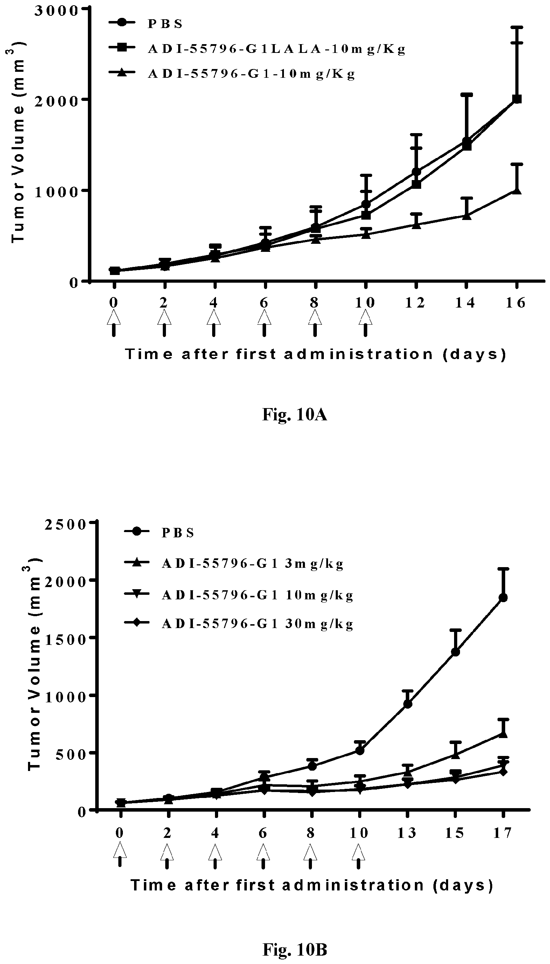

Specifically, CT-26 tumor-bearing mouse models were first established by subcutaneous inoculation. When the average tumor volume reached 100 to 200 mm³, the animals were divided into groups. Different doses of anti-TIGIT antibody of the present invention were administered intraperitoneally for treatment, and the changes in tumor volume and body weight were monitored in the mice of each group at a monitoring frequency of once per 2 to 3 days, for 2 to 3 weeks. The dosage and method of administration were shown in Tables 4 to 5. The results were shown in Figs. 10A to 10B. The results showed that the anti-TIGIT antibody ADI-55796-G1 of the present invention could significantly inhibit the growth of tumors in mouse in a dose-dependent manner.

**Table 4: Experimental protocol for tumor inhibitory activity of anti-TIGIT antibody**

| Group | Administration dose | Administration frequency |
|---|---|---|
| PBS | N/A | 6 |
| ADI-55796-G1 | 10 mg/kg | 6 |
| ADI-55796-G1LALA | 10 mg/kg | 6 |

**Table 5: Experimental protocol for dose-dependent tumor inhibitory activity of anti-TIGIT antibody**

| Group | Administration dose | Administration frequency |
|---|---|---|
| PBS | N/A | 6 |
| ADI-55796-G1 | 3 mg/kg | 6 |
| ADI-55796-G1 | 10 mg/kg | 6 |
| ADI-55796-G1 | 30 mg/kg | 6 |

### Example 9: In vivo synergistic pharmacodynamics study on anti-TIGIT antibody and anti-PD-L1 antibody in wild-type Balb/c mice

In this experiment, CT-26 tumor cells were inoculated into wild-type Balb/c mice to determine the synergistic anti-tumor effect of the anti-TIGIT antibody of the present invention and the anti-PD-L1 antibody.

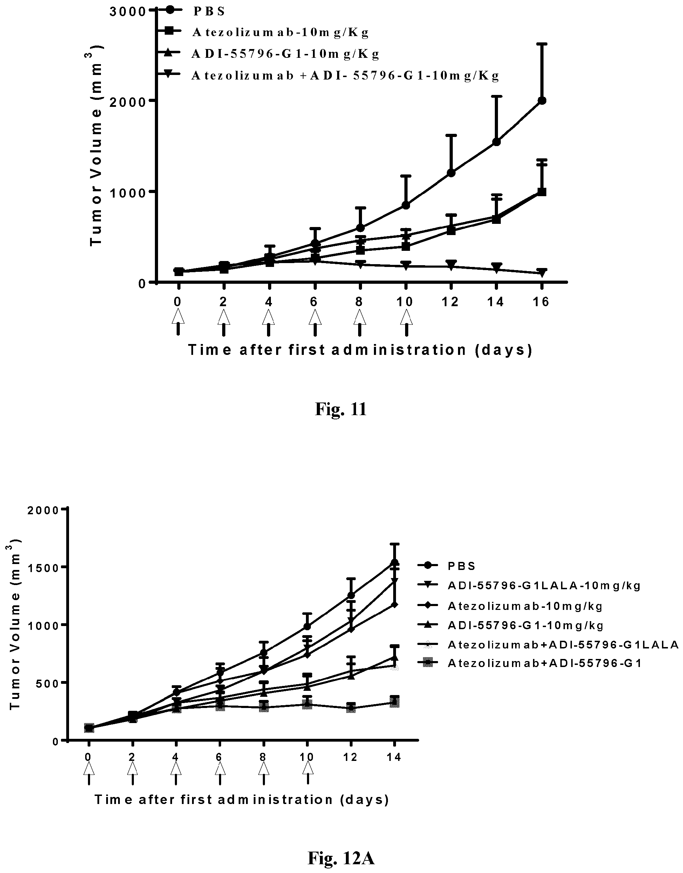

Specifically, CT-26 cell tumor-bearing mouse models were first established by subcutaneous inoculation. When the average tumor volume reached 100 to 200 mm³, the animals were divided into groups, and the anti-TIGIT antibody of the present invention and/or anti-PD-L1 antibody (Atezolizumab) were administered intraperitoneally for treatment. The changes in tumor volume and body weight of the mice in each group were monitored at a monitoring frequency of once per 2 to 3 days, for 2 to 3 weeks. The dosage and method of administration were shown in Table 6. The results were shown in Fig. 11. The results showed that the anti-TIGIT antibody ADI-55796-G1 of the present invention in combination with the anti-PD-L1 antibody Atezolizumab showed significantly better tumor inhibitory activity than two corresponding monoclonal antibodies. It was suggested that the anti-TIGIT antibody ADI-55796-G1 of the present invention could exert synergistic anti-tumor activity with the anti-PD-L1 antibody Atezolizumab in the CT-26 tumor-bearing wild-type mouse model.

**Table 6: Administration regimen of anti-TIGIT antibody and anti-PD-L1 antibody**

| Group | Administration dose | Administration frequency |
|---|---|---|
| PBS | N/A | 6 |
| Atezolizumab | 10mg/kg | 6 |
| ADI-55796-G1 | 10mg/kg | 6 |
| Atezolizumab+ADI-55796-G1 | 10mg/kg+10 mg/kg | 6 |

### Example 10: In vivo synergistic pharmacodynamics study on anti-TIGIT antibody and anti-PD-L1 antibody in huTIGIT KI mice

In this experiment, CT-26 tumor cells were transplanted into human TIGIT-knock-in transgenic mice (huTIGIT KI mice) to determine the synergistic anti-tumor effect of the TIGIT antibody of the present invention and the anti-PD-L1 antibody.

Specifically, CT-26 tumor-bearing mouse model was first established by subcutaneous inoculation. When the average tumor volume reached 80 to 120 mm³, the animals were divided into groups. Different antibodies at different doses were administrated by intraperitoneal injection, and the changes in tumor volume and body weight of the mice in each group were monitored at a monitoring frequency of once per 2 to 3 days, for 2 to 3 weeks. The dosage and method of administration were shown in Tables 7 to 8. The results were shown in Figs. 12A to 12B. The results showed that the anti-TIGIT antibodies ADI-55796-G1, ADI-55796-G1LALA, and ADI-55812-G1 of the present invention were all observed to have synergistic antitumor activity with the anti-PD-L1 antibody Atezolizuamb in the human TIGIT-konck-in transgenic mouse model transplanted with CT-26 tumor cells.

**Table 7: Dosage regimen of anti-TIGIT antibody and anti-PD-L1 antibody (corresponding to Fig. 12A)**

| Group | Administration dose | Administration frequency |
|---|---|---|
| PBS | N/A | 6 |
| ADI-55796-G1LALA | 10mg/kg | 6 |
| Atezolizumab | 10mg/kg | 6 |
| ADI-55796-G1 | 10mg/kg | 6 |
| Atezolizumab+ADI-55796-G1LALA | 10mg/kg+10 mg/kg | 6 |
| Atezolizumab+ADI-55796-G1 | 10mg/kg+10 mg/kg | 6 |

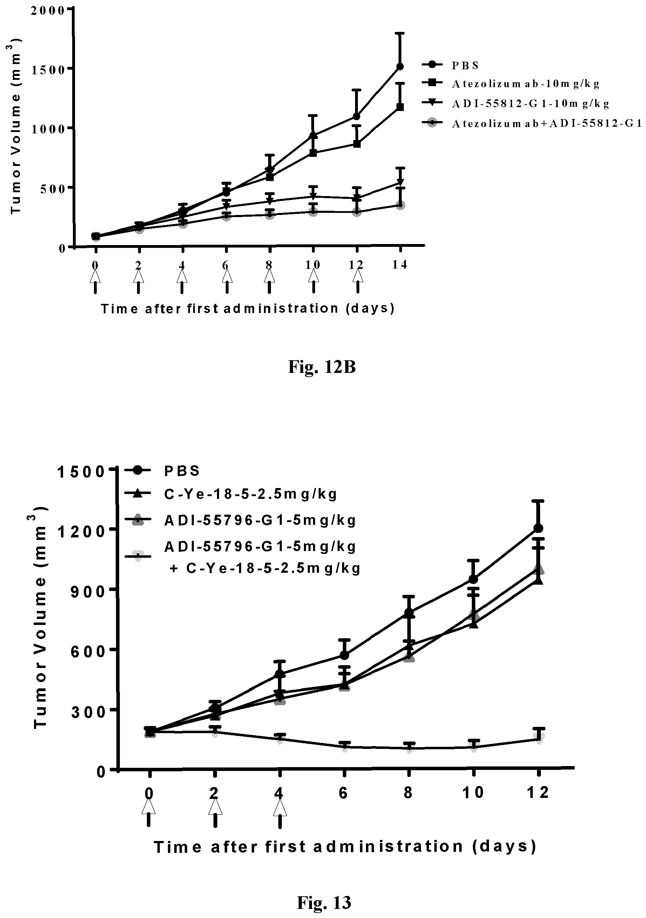

**Table 8: Dosage regimen of anti-TIGIT antibody and anti-PD-L1 antibody (corresponding to Fig. 12B)**

| Group | Administration dose | Administration frequency |
|---|---|---|
| PBS | N/A | 7 |
| Atezolizumab | 10mg/kg | 7 |
| ADI-55812-G1 | 10mg/kg | 7 |
| Atezolizumab+ADI-55812-G1 | 10mg/kg+10 mg/kg | 7 |

### Example 11: In vivo synergistic pharmacodynamics study on anti-TIGIT antibody and anti-PD-L1 antibody in B-NDG mice inoculated with mixed A375 and human PBMC

In this experiment, B-NDG mice were inoculated with mixed A375 (purchased from Addexbio, Cat. No.: C0020004) and human PBMC cells (Milestone^{®} Biotechnologies, A10S033014/PB100C) to determine the synergistic antitumor activity of the TIGIT antibody of the present invention and the anti-PD-L1 antibody.

Specifically, A375 and human PBMC tumor-bearing mouse models were first established by subcutaneous inoculation of mixed A375 and human PBMC cells. When the average tumor volume reached about 200 mm³, the animals were divided into groups. Different antibodies at different doses were administered intraperitoneally, and the changes in tumor volume and body weight of mice in each group were monitored at a monitoring frequency of once per 2 to 3 days, for 2 to 3 weeks. The dosage and method of administration were shown in Table 9. The results were shown in Fig. 13. The results showed that the combined administration of the anti-TIGIT antibody ADI-55796-G1 of the present invention and the anti-PD-L1 monoclonal antibody C-Ye-18-5 (see, PCT patent application: PCT/IB2020/058303) showed significant synergistic effect.

**Table 9: Synergistic dosage regimen of anti-TIGIT antibody and anti-PD-L1 antibody in A375 tumor-bearing model**

| Group | Administration dose | Administration frequency |
|---|---|---|
| PBS | N/A | 3 |
| ADI-55796-G1 | 5 mg/kg | 3 |
| C-Ye-18-5 | 2.5 mg/kg | 3 |
| C-Ye-18-5+ADI-55796-G1 | 2.5 mg/kg+5 mg/kg | 3 |

### Example 12: Study on in vivo half-life of anti-TIGIT antibody in mice

The in vivo half-life of the anti-TIGIT antibody of the present invention in mice was detected by a single tail vein injection method.

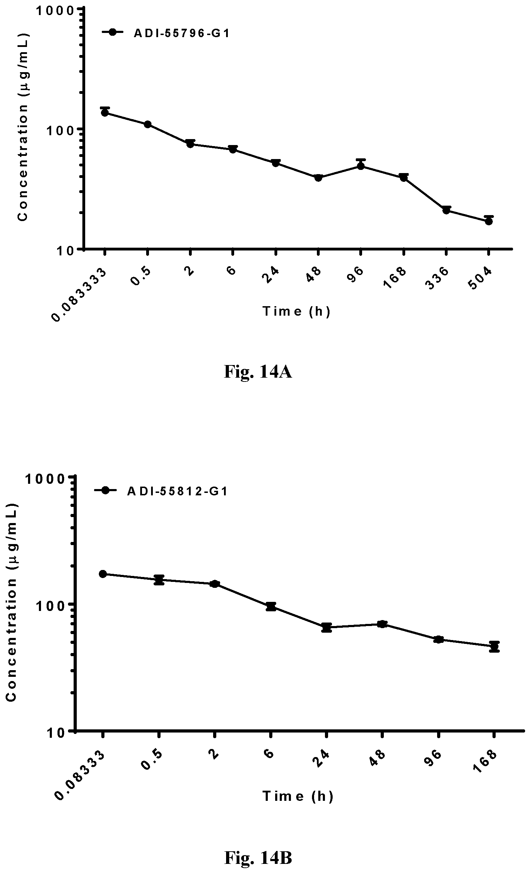

Specifically, Balb/c mice were used in this experiment, half being male and the other half being female, raised with 12/12 hours of light/dark regulation, at a temperature of 24±2°C, a humidity of 40-70%, and free access to water and food. On the day of the experiment, the Balb/c mice were given a single tail vein injection of monoclonal antibody molecules at a dose of 10 mg/kg. Blood collection time points: 5 minutes, 0.5 hours, 2 hours, 6 hours, 24 hours, 48 hours, 96 hours, 168 hours, 336 hours, and 504 hours after administration, wherein blood was collected from mouse orbits. Whole blood samples were allowed to stand at 2-8°C for 30 minutes, and centrifuged at 12,000 rpm for 5 minutes to collect serum. The resulting serum was then centrifuged at 2-8°C, 12,000 rpm for 5 minutes, and stored at -80°C. The content of monoclonal antibody molecules in the serum was detected by ELISA. The results were shown in Figs. 14A to 14B. The results showed that the in vivo half-lives of the anti-TIGIT antibodies ADI-55796-G1 and ADI-55812-G1 of the present invention in mice after a single injection were 257 hours and 249 hours, respectively.

Although the specific embodiments of the present invention have been described in detail, those skilled in the art will understand that various modifications and changes can be made to the details based on all teachings that have been published, and these changes are within the protection scope of the present invention. The full scope of the present invention is given by the appended claims and any equivalents thereof.

## Claims

1. An antibody or antigen-binding fragment thereof capable of specifically binding to TIGIT, wherein the antibody or antigen-binding fragment thereof comprises:
(a) a VH CDR1 or variant thereof, a VH CDR2 or variant thereof and a VH CDR3 or variant thereof comprised in the heavy chain variable region (VH) as set forth in SEQ ID NO: 4; and/or, a VL CDR1 or variant thereof, a VL CDR2 or variant thereof, and a VL CDR3 or variant thereof comprised in the light chain variable region (VL) as set forth in SEQ ID NO: 8; or,
(b) a VH CDR1 or variant thereof, a VH CDR2 or variant thereof and a VH CDR3 or variant thereof comprised in the heavy chain variable region (VH) as set forth in SEQ ID NO: 12; and/or, a VL CDR1 or variant thereof, a VL CDR2 or variant thereof, and a VL CDR3 or variant thereof comprised in the light chain variable region (VL) as set forth in SEQ ID NO: 16;
wherein, the variant has a substitution, deletion or addition of one or several amino acids (e.g., a substitution (such as conservative substitution), deletion or addition of 1, 2 or 3 amino acids) as compared to the sequence from which it is derived; preferably, the substitution is a conservative substitution;
preferably, the antibody or antigen-binding fragment thereof comprises:
(a) three CDRs comprised in the heavy chain variable region (VH) as set forth in SEQ ID NO: 4; and/or, three CDRs comprised in the light chain variable region (VL) as set forth in SEQ ID NO: 8; or,
(b) three CDRs comprised in the heavy chain variable region (VH) as set forth in SEQ ID NO: 12; and/or, three CDRs comprised in the light chain variable region (VL) as set forth in SEQ ID NO: 16;
preferably, the three CDRs comprised in the VH and/or the three CDRs comprised in the VL are defined by the Kabat, IMGT or Chothia numbering system.

2. The antibody or antigen-binding fragment thereof according to claim 1, comprising:
(a) the following three complementarity-determining regions (CDRs) of the heavy chain variable region (VH): a VH CDR1 having the sequence as set forth in SEQ ID NO: 1, a VH CDR2 having the sequence as set forth in SEQ ID NO: 2, and a VH CDR3 having the sequence as set forth in SEQ ID NO: 3; and/or, the following three complementarity-determining regions (CDRs) of the light chain variable region (VL): a VL CDR1 having the sequence as set forth in SEQ ID NO: 5, a VL CDR2 having the sequence as set forth in SEQ ID NO: 6, a VL CDR3 having the sequence as set forth in SEQ ID NO: 7; or,
(b) the following three complementarity-determining regions (CDRs) of the heavy chain variable region (VH): a VH CDR1 having the sequence as set forth in SEQ ID NO: 9, a VH CDR2 having the sequence as set forth in SEQ ID NO: 10, and a VH CDR3 having the sequence as set forth in SEQ ID NO: 11; and/or, the following three complementarity-determining regions (CDRs) of the light chain variable region (VL): a VL CDR1 having the sequence as set forth in SEQ ID NO: 13, a VL CDR2 having the sequence as set forth in SEQ ID NO: 14, a VL CDR3 having the sequence as set forth in SEQ ID NO: 15;
wherein, the CDR is defined by the IMGT numbering system.

3. The antibody or antigen-binding fragment thereof according to claim 1 or 2, wherein the antibody or antigen-binding fragment thereof further comprises a framework region of a human immunoglobulin;
for example, the antibody or antigen-binding fragment thereof comprises a framework region comprised in an amino acid sequence encoded by a human antibody germline gene; for example, the antibody or antigen-binding fragment thereof comprises: a heavy chain framework region comprised in an amino acid sequence encoded by a human heavy chain germline gene, and/or a light chain framework region comprised in an amino acid sequence encoded by a human light chain germline gene.

4. The antibody or antigen-binding fragment thereof according to any one of claims 1 to 3, which comprises:
(a) a VH comprising the sequence as set forth in SEQ ID NO: 4 or variant thereof, and/or, a VL comprising the sequence as set forth in SEQ ID NO: 8 or variant thereof; or,
(b) a VH comprising the sequence as set forth in SEQ ID NO: 12 or variant thereof, and/or a VL comprising the sequence as set forth in SEQ ID NO: 16 or variant thereof;
wherein, the variant has a substitution, deletion or addition of one or several amino acids (e.g., a substitution, deletion or addition of 1, 2, 3, 4 or 5 amino acids), or has a sequence identity of at least 80%, at least 85%, at least 90%, at least 91%, at least 92%, at least 93%, at least 94%, at least 95%, at least 96%, at least 97%, at least 98%, at least 99% or 100%, as compared to the sequence from which it is derived; preferably, the substitution is a conservative substitution;
for example, the antibody or antigen-binding fragment thereof comprises a VH as set forth in SEQ ID NO: 4, and/or a VL as set forth in SEQ ID NO: 8;
for example, the antibody or antigen-binding fragment thereof comprises a VH as set forth in SEQ ID NO: 12, and/or a VL as set forth in SEQ ID NO: 16.

5. The antibody or antigen-binding fragment thereof according to any one of claims 1 to 4, which further comprises a constant region derived from a human immunoglobulin;
preferably, the heavy chain of the antibody or antigen-binding fragment thereof comprises a heavy chain constant region derived from a human immunoglobulin (e.g., IgG1, IgG2, IgG3 or IgG4); preferably, the antibody or antigen-binding fragment thereof possesses ADCC activity; preferably, the antibody or antigen-binding fragment thereof comprises a mutated or chemically modified Fc region that provides increased ADCC activity compared to a wild-type Fc region; preferably, the antibody or antigen-binding fragment thereof has low or no fucosylation;
preferably, the light chain of the antibody or antigen-binding fragment thereof comprises a light chain constant region (e.g., a constant region of κ or λ chain) derived from a human immunoglobulin.

6. The antibody or antigen-binding fragment thereof according to any one of claims 1 to 5, wherein the antigen-binding fragment is selected from the group consisting of Fab, Fab', (Fab')₂, Fv, disulfide-linked Fv, scFv, diabody and single domain antibody (sdAb); and/or, the antibody is a chimeric antibody, bispecific antibody or multispecific antibody.

7. An isolated nucleic acid molecule, which encodes the antibody or antigen-binding fragment thereof according to any one of claims 1 to 6, or its heavy chain variable region and/or light chain variable region.

8. A vector, which comprises the nucleic acid molecule according to claim 7; preferably, the vector is a cloning vector or an expression vector.

9. A host cell, which comprises the nucleic acid molecule according to claim 7 or the vector according to claim 8;
preferably, the host cell has low or no fucosylation activity, for example is a mammalian cell (e.g., a CHO cell) lacking a gene encoding a fucosyltransferase.

10. A method for preparing the antibody or antigen-binding fragment thereof according to any one of claims 1 to 6, comprising culturing the host cell according to claim 9 under a condition that allows the expression of the antibody or antigen-binding fragment thereof, and recovering the antibody or antigen-binding fragment thereof from a cell culture of the host cell;
preferably, the host cell has low or no fucosylation activity, for example, is a mammalian cell (e.g., a CHO cell) lacking a gene encoding a fucosyltransferase.

11. A pharmaceutical composition, which comprises the antibody or antigen-binding fragment thereof according to any one of claims 1 to 6, the isolated nucleic acid molecule according to claim 7, the vector according to claim 8 or the host cell according to claim 9, and a pharmaceutically acceptable carrier and/or excipient;
preferably, the pharmaceutical composition further comprises an additional immune checkpoint inhibitor;
preferably, the pharmaceutical composition further comprises an anti-PD-1 antibody or an anti-PD-L1 antibody.

12. Use of the antibody or antigen-binding fragment thereof according to any one of claims 1 to 6, the isolated nucleic acid molecule according to claim 7, the vector according to claim 8, the host cell according to claim 9, or the pharmaceutical composition according to claim 11 in the manufacture of a medicament, wherein the medicament is used for:
(1) increasing an immune cell activity in vitro or in a subject (e.g., a human);
(2) enhancing an immune response in a subject (e.g., a human);
(3) preventing and/or treating a tumor in a subject (e.g., a human); or
(4) preventing and/treating an infection in a subject (e.g., a human).
preferably, the immune cell is a T cell and/or NK cell;
preferably, the immune response is a T cell- or NK cell-mediated immune response; preferably, the tumor involves a TIGIT-positive infiltrating T cell and/or NK cell, and/or involves a TIGIT ligand (e.g., CD155 and/or CD112)-positive tumor cell;
preferably, the tumor is selected from solid tumor or hematological tumor (e.g., leukemia, lymphoma, myeloma);
preferably, the tumor is selected from the group consisting of colorectal cancer, colon cancer, bladder cancer, breast cancer, uterine/cervical cancer, ovarian cancer, prostate cancer, testicular cancer, esophageal cancer, gastrointestinal cancer, pancreatic cancer, kidney cancer, head and neck cancer, lung cancer, gastric cancer, germ cell cancer, bone cancer, liver cancer, thyroid cancer, skin cancer, central nervous system tumor, lymphoma, leukemia, myeloma, sarcoma, and melanoma;
preferably, the infection is selected from the group consisting of viral infection, bacterial infection, fungal infection and parasitic infection;
preferably, the subject is a mammal, such as a human, a cynomolgus monkey or a mouse;
preferably, the antibody or antigen-binding fragment thereof, the isolated nucleic acid molecule, the vector, the host cell or the pharmaceutical composition is used alone or in combination with an additional pharmaceutically active agent; preferably, the additional pharmaceutically active agent is an additional immune checkpoint inhibitor;
preferably, the antibody or antigen-binding fragment thereof, the isolated nucleic acid molecule, the vector, the host cell or the pharmaceutical composition is used in combination with an anti-PD-1 antibody or an anti-PD-L1 antibody.

13. A method for enhancing an immune response or preventing and/or treating a tumor or infection in a subject, comprising: administering to a subject in need thereof an effective amount of the antibody or antigen-binding fragment thereof according to any one of claims 1 to 6, or the pharmaceutical composition according to claim 11;
preferably, the tumor involves a TIGIT-positive infiltrating T cell and/or NK cell, and/or involves a TIGIT ligand (e.g., CD155 and/or CD112)-positive tumor cell;
preferably, the tumor is selected from solid tumor or hematological tumor (e.g., leukemia, lymphoma, myeloma);
preferably, the tumor is selected from the group consisting of colorectal cancer, colon cancer, bladder cancer, breast cancer, uterine/cervical cancer, ovarian cancer, prostate cancer, testicular cancer, esophageal cancer, gastrointestinal cancer, pancreatic cancer, kidney cancer, head and neck cancer, lung cancer, gastric cancer, germ cell cancer, bone cancer, liver cancer, thyroid cancer, skin cancer, central nervous system tumor, lymphoma, leukemia, myeloma, sarcoma, and melanoma;
preferably, the infection is selected from the group consisting of viral infection, bacterial infection, fungal infection and parasitic infection;
preferably, the subject is a mammal, such as a human.

14. A conjugate, which comprises the antibody or antigen-binding fragment thereof according to any one of claims 1 to 6, and a detectable label connected to the antibody or antigen-binding fragment thereof;
preferably, the detectable label is selected from enzyme (e.g., horseradish peroxidase or alkaline phosphatase), chemiluminescent reagent (e.g., acridinium ester, luminol and derivative thereof, or ruthenium derivative), fluorescent dye (e.g., fluorescein or fluorescent protein), radionuclide or biotin.

15. A kit, which comprises the antibody or antigen-binding fragment thereof according to any one of claims 1 to 6 or the conjugate according to claim 14;
preferably, the kit comprises the conjugate according to claim 14;
preferably, the kit comprises the antibody or antigen-binding fragment thereof according to any one of claims 1 to 6, and a second antibody that specifically recognizes the antibody or antigen-binding fragment thereof; optionally, the second antibodies further comprises a detectable label such as enzyme (e.g., horseradish peroxidase or alkaline phosphatase), chemiluminescent reagent (e.g., acridinium ester, luminol and derivative thereof, or ruthenium derivative), fluorescent dye (e.g., fluorescein or fluorescent protein), radionuclide or biotin.

16. A method for detecting the presence or level of TIGIT in a sample, comprising using the antibody or antigen-binding fragment thereof according to any one of claims 1 to 6 or the conjugate according to claim 14;
preferably, the method is an immunoassay, such as an immunoblotting assay, an enzyme immunoassay (e.g., ELISA), a chemiluminescence immunoassay, a fluorescent immunoassay or a radioimmunoassay;
preferably, the method comprises using the conjugate according to claim 14;
preferably, the method comprises using the antibody or antigen-binding fragment thereof according to any one of claims 1 to 6, and the method further comprises using a second antibody carrying a detectable label (e.g., an enzyme (e.g., horseradish peroxidase or alkaline phosphatase), a chemiluminescent reagent (e.g., acridinium ester, luminol and derivative thereof, or ruthenium derivative), a fluorescent dye (e.g., fluorescein or fluorescent protein), a radionuclide or a biotin) to detect the antibody or antigen-binding fragment thereof.

17. Use of the antibody or antigen-binding fragment thereof according to any one of claims 1 to 6 or the conjugate according to claim 14 in the manufacture of a detection reagent, wherein the detection reagent is used to detect the presence or level of TIGIT in a sample;
preferably, the detection reagent detects the presence or level of TIGIT in the sample by the method according to claim 16;
preferably, the sample is a cell sample (e.g., an immune cell) from a subject (e.g., a mammal, preferably a human, a cynomolgus monkey or a mouse).
